# EUROPEAN PATENT SPECIFICATION

(11) **EP 3 016 685 B1**
(45) Date of publication and mention of the grant of the patent: **31.01.2018**
(21) Application number: 14820560.2
(22) Date of filing: 01.07.2014
(51) Int. Cl.: A61K 39/40, A61K 39/395, A61K 39/104, C07H 1/00, C12N 1/20, C07H 15/04, C07K 16/44

(54) **SYNTHETIC OLIGOSACCHARIDES FOR P. AERUGINOSA VACCINE**
SYNTHETISCHE OLIGOSACCHARIDE FÜR P. AERUGINOSA-IMPFSTOFF
OLIGOSACCHARIDES DE SYNTHÈSE POUR VACCIN CONTRE P. AERUGINOSA

(30) Priority: 03.07.2013 US 201361842474 P
(43) Date of publication of application: 11.05.2016
(73) Proprietor: Visterra, Inc., Cambridge, MA 02139 (US)
(72) Inventor: CAMPBELL, A. Stewart, Framingham, MA 01701 (US); PLANTE, Obadiah, J., Danvers, MA 01923 (US)
(74) Representative: HGF Limited
(86) International application number: PCT/US2014/045057
(87) International publication number: WO 2015/002954

(56) References cited:
- EP-A1- 2 554 549
- WO-A1-2012/082635
- WO-A1-2014/014670
- WO-A2-2011/133227
- BOZHENA S. KOMAROVA ET AL: "First Synthesis of Pentasaccharide Glycoform I of the Outer Core Region of the Pseudomonas aeruginosa Lipopolysaccharide", THE JOURNAL OF ORGANIC CHEMISTRY, vol. 73, no. 21, 7 November 2008 (2008-11-07), pages 8411-8421, XP055255592, US ISSN: 0022-3263, DOI: 10.1021/jo801561p
- BOZHENA S. KOMAROVA ET AL: "Synthesis of pentasaccharides corresponding to the glycoform II of the outer core region of the Pseudomonas aeruginosa lipopolysaccharide", CARBOHYDRATE RESEARCH, vol. 360, 1 October 2012 (2012-10-01), pages 56-68, XP055308998, GB ISSN: 0008-6215, DOI: 10.1016/j.carres.2012.07.019
- W. BITTER ET AL: "Species-Specific Functioning of the Pseudomonas XcpQ Secretin: Role for the C-Terminal Homology Domain and Lipopolysaccharide", JOURNAL OF BACTERIOLOGY, vol. 189, no. 8, 15 April 2007 (2007-04-15), pages 2967-2975, XP055309013, US ISSN: 0021-9193, DOI: 10.1128/JB.01583-06
- OLGA V. BYSTROVA ET AL: "Structural studies on the core and the O-polysaccharide repeating unit of Pseudomonas aeruginosa immunotype 1 lipopolysaccharide", EUROPEAN JOURNAL OF BIOCHEMISTRY, vol. 269, no. 8, 26 April 2002 (2002-04-26), pages 2194-2203, XP055309022, GB ISSN: 0014-2956, DOI: 10.1046/j.1432-1033.2002.02875.x
- BYSTROVA O V ET AL: "Structure of the biological repeating unit of the O-antigen of Pseudomonas aeruginosa immunotype 4 containing both 2-acetamido-2,6-dideoxy-d-glucose and 2-acetamido-2,6-dideoxy-d-galactose", CARBOHYDRATE RESEARCH, PERGAMON, GB, vol. 338, no. 17, 12 August 2003 (2003-08-12), pages 1801-1806, XP004442158, ISSN: 0008-6215, DOI: 10.1016/S0008-6215(03)00262-3
- CHI-JEN ET AL: "Bacterial capsular polysaccharides-biochemistry, immunity and vaccine", MOLECULAR IMMUNOLOGY, PERGAMON, GB, vol. 24, no. 10, 1 October 1987 (1987-10-01), pages 1005-1019, XP023795347, ISSN: 0161-5890, DOI: 10.1016/0161-5890(87)90067-8 [retrieved on 1987-10-01]
- MATTHIAS A. OBERLI ET AL: "A Possible Oligosaccharide-Conjugate Vaccine Candidate for Clostridium difficile Is Antigenic and Immunogenic", CHEMISTRY & BIOLOGY, vol. 18, no. 5, 1 May 2011 (2011-05-01), pages 580-588, XP055007928, ISSN: 1074-5521, DOI: 10.1016/j.chembiol.2011.03.009
- BYSTROVA, OV ET AL.: 'Full Structure Of The Lipopolysaccharide Of Pseudomonas aeruginosa Immunotype 5.' BIOCHEMISTRY vol. 69, no. 2, February 2004, MOSCOW, pages 170 - 175, XP055184549

## Description

### FIELD OF THE INVENTION

The present invention relates to immunogenic and immunoprotective compositions and methods for making and using homogenous synthetic *P. aeruginosa* lipooligosaccharide (LOS)-based oligosaccharides, conjugates, and antibodies derived therefrom.

### SUMMARY

Outer membrane oligosaccharide core structures isolated from *P. aeruginosa* have been previously reported, e.g. by Komarova et al., J.Org. Chem. 2008 73(21) :8411-21, Komarova et al., Carbohydr. Res. 2012, 360:56-68, Bitter et al. J. Bacteriol. 2007, 189(8) : 2967-75, Bystrova et al., Eur. J. Biochem., 2002, 269(8): 2194-203, Bystrova et al., Carbohydr. Res., 2003, 338(17): 1801-6.

The present invention provides synthetic oligosaccharide **1a** or **1b:** 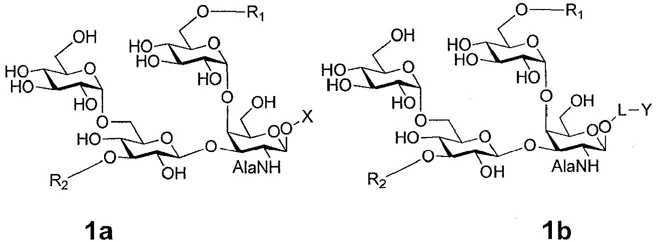 where each of R¹ and R² is independently H, a monosaccharide or an oligosaccharide, X is H, a linker group, or a protecting group; L is a linker and Y is a carrier. In some embodiments, R¹ and R² are each independently H, α-Rha-, α-Glc(1-2)-α-Rha-, β-QuiNAc(1-3)-α-Rha-, β-FucNAc(1-3)-α-Rha-, α-Rha[2,3,4-OAc]-, β-QuiNAc(1-3)-α-Rha[2,4-OAc]-, or β-FucNAc(1-3)-α-Rha[2,4,-OAc]-. In some embodiments, R¹ and R² are selected from the combinations in the following table:

| **R¹** | **R²** |
|---|---|
| α-Rha- | H |
| α-Glc(1-2)-α-Rha- | H |
| H | α-Rha- |
| H | β-QuiNAc(1-3)-α-Rha- |
| H | β-FucNAc(1-3)-α-Rha- |
| H | α-Rha[2,3,4-OAc]- |
| H | β-QuiNAc(1-3)-α-Rha[2,4-OAc]- |
| H | β-FucNAc(1-3)-α-Rha[2,4,-OAc]- |

The present invention also includes compositions comprising an antigen **1a** and/or **1b** and a pharmaceutically acceptable vehicle. Preferably the composition contains a single antigen or a known, defined mixture of antigens.

The invention further provides vaccine compositions, including immunogenic and immunoprotective compositions, comprising antigen **1a** and/or **1b** and a pharmaceutically acceptable vehicle. These vaccine compositions can optionally include a pharmaceutically acceptable adjuvant. Preferably, the vaccine compositions are endotoxin-free. The vaccine compositions can be mono-, di-, tri- or tetravalent.

The invention further provides a method for synthetically forming oligosaccharides **1a** and antigens **1b.**

The invention further provides methods for diagnosing, treating, and preventing infections caused by *P. aeruginosa.*

### DETAILED DESCRIPTION

### Definitions

In order to provide a clear and consistent understanding of the specification and claims, the following definitions are provided.

Units, prefixes, and symbols may be denoted in their SI accepted form. Numeric ranges recited herein are inclusive of the numbers defining the range and include and are supportive of each integer within the defined range. Unless otherwise noted, the terms "a" or "an" are to be construed as meaning "at least one of." The section headings used herein are for organizational purposes only and are not to be construed as limiting the subject matter described.

As used herein, "oligosaccharide" refers to a compound containing two or more monosaccharide units. Oligosaccharides are considered to have a reducing end and a non-reducing end, whether or not the monosaccharide unit at the reducing end is in fact a reducing sugar. In accordance with accepted nomenclature, oligosaccharides are depicted herein with the non-reducing end on the left and the reducing end on the right. All oligosaccharides described herein are described with the name or abbreviation for the non-reducing monosaccharide (e.g., Gal), preceded by the configuration of the glycosidic bond (α or β), the ring bond, the ring position of the reducing monosaccharide involved in the bond, and then the name or abbreviation of the reducing monosaccharide (e.g., GlcNAc). The linkage between two sugars may be expressed, for example, as 2,3, 2→3, or 2-3. Each monosaccharide is a pyranose or furanose.

As used herein, "monosaccharide" or "monosaccharide unit" refers to a single sugar residue in an oligosaccharide, including derivatives therefrom. Within the context of an oligosaccharide, an individual monomer unit is a monosaccharide which is (or can be) bound through a hydroxyl group to another monosaccharide.

As used herein, "endotoxin-free" refers to an oligosaccharide that does not contain endotoxins or endotoxin components normally present in isolated bacterial carbohydrates and polysaccharides.

As used herein, "synthetic" refers to material which is substantially or essentially free from components, such as endotoxins, glycolipids, unrelated oligosaccharides, etc., which normally accompany a compound when it is isolated. Typically, synthetic compounds are at least about 90% pure, usually at least about 95%, and preferably at least about 99% pure. Purity can be indicated by a number of means well known in the art. Preferably, purity is measured by HPLC. The identity of the synthetic material can be determined by mass spectroscopy and/or NMR spectroscopy.

As used herein the term "linker" refers to either a bond or a moiety which at one end exhibits a grouping able to enter into a covalent bonding with a reactive functional group of the carrier, e.g. an amino, thiol, or carboxyl group, and at the other end a grouping likewise able to enter into a covalent bonding with a hydroxyl group or an amino group of an oligosaccharide according to the present invention. Between the two functional groups of the linker molecule there is a biocompatible bridging molecule of suitable length, e.g. substituted or unsubstituted heteroalkylene, arylalkylene, alkylene, alkenylene, or (oligo)alkylene glycol groups. Linkers preferably include a substituted or unsubstituted (C₁-C₁₀) alkylene group or a substituted or unsubstituted (C₂-C₁₀) alkenylene group.

As used herein, the term "carrier" refers to a protein, peptide, lipid, polymer, dendrimer, virosome, virus-like particle (VLP), or combination thereof, which is coupled to the oligosaccharide to enhance the immunogenicity of the resulting oligosaccharide-carrier conjugate to a greater degree than the oligosaccharide alone.

As used herein, "protein carrier" refers to a protein, peptide or fragment thereof, which is coupled or conjugated to an oligosaccharide to enhance the immunogenicity of the resulting oligosaccharide-protein carrier conjugate to a greater degree than the oligosaccharide alone. For example, when used as a carrier, the protein carrier may serve as a T-dependent antigen which can activate and recruit T-cells and thereby augment T-cell dependent antibody production.

As used herein, "conjugated" refers to a chemical linkage, either covalent or non-covalent, that proximally associates an oligosaccharide with a carrier so that the oligosaccharide conjugate has increased immunogenicity relative to an unconjugated oligosaccharide.

As used herein, "conjugate" refers to an oligosaccharide chemically coupled to a carrier through a linker and/or a cross-linking agent.

As used herein, "passive immunity" refers to the administration of antibodies to a subject, whereby the antibodies are produced in a different subject (including subjects of the same and different species) such that the antibodies attach to the surface of the bacteria and cause the bacteria to be phagocytosed or killed.

As used herein, "protective immunity" means that a vaccine or immunization schedule that is administered to a animal induces an immune response that prevents, retards the development of, or reduces the severity of a disease that is caused by a pathogen or diminishes or altogether eliminates the symptoms of the disease. Protective immunity may be predicted based on the ability of serum antibody to activate complement-mediated bactericidal activity or confer passive protection against a bacterial infection in a suitable animal challenge model.

As used herein, "immunoprotective composition" refers to a composition formulated to provide protective immunity in a host.

As used herein, "in a sufficient amount to elicit an immune response" or "in an effective amount to stimulate an immune response" (e.g., to epitopes present in a preparation) means that there is a detectable difference between an immune response indicator measured before and after administration of a particular antigen preparation. Immune response indicators include but are not limited to: antibody titer or specificity, as detected by an assay such as enzyme-linked immunoassay (ELISA), bactericidal assay (e.g., to detect serum bactericidal antibodies), flow cytometry, immunoprecipitation, Ouchter-Lowry immunodiffusion; binding detection assays of, for example, spot, Western blot or antigen arrays; cytotoxicity assays, and the like.

As used herein, "antibody" encompasses polyclonal and monoclonal antibody preparations, as well as preparations including hybrid antibodies, altered antibodies, F(ab')² fragments, F(ab) molecules, Fv fragments, single chain fragment variable displayed on phage (scFv), single domain antibodies, chimeric antibodies, humanized antibodies, and functional fragments thereof which exhibit immunological binding properties of the parent antibody molecule.

As used herein, "monoclonal antibody" refers to an antibody composition having a homogeneous antibody population. The term is not limited by the manner in which it is made. The term encompasses whole immunoglobulin molecules, as well as Fab molecules, F(ab')₂ fragments, Fv fragments, single chain fragment variable displayed on phage (scFv), and other molecules that exhibit immunological binding properties of the parent monoclonal antibody molecule.

As used herein, "specifically binds to an antibody" or "specifically immunoreactive with", when referring to an oligosaccharide, protein or peptide, refers to a binding reaction which is based on and/or is probative of the presence of the antigen in a sample which may also include a heterogeneous population of other molecules. Thus, under designated immunoassay conditions, the specified antibody or antibodies bind(s) to a particular antigen or antigens in a sample and does not bind in a significant amount to other molecules present in the sample. Specific binding to an antibody under such conditions may require an antibody or antiserum that is selected for its specificity for a particular antigen or antigens.

As used herein, "antigen" refers to any substance that may be specifically bound by an antibody molecule.

As used herein, "immunogen" and "immunogenic composition" refer to an antigenic composition capable of initiating lymphocyte activation resulting in an antigen-specific immune response.

As used herein, "epitope" refers to a site on an antigen to which specific B cells and/or T cells respond. The term is also used interchangeably with "antigenic determinant" or "antigenic determinant site." B cell epitope sites on proteins, oligosaccharides, or other biopolymers may be composed of moieties from different parts of the macromolecule that have been brought together by folding. Epitopes of this kind are referred to as conformational or discontinuous epitopes, since the site is composed of segments of the polymer that are discontinuous in the linear sequence but are continuous in the folded conformation(s). Epitopes that are composed of single segments of biopolymers or other molecules are termed continuous or linear epitopes. T cell epitopes are generally restricted to linear peptides. Antibodies that recognize the same epitope can be identified in a simple immunoassay showing the ability of one antibody to block the binding of another antibody to a target antigen.

The term Ac means acetyl (-C(O)CH₃).

The term TBS means tert-butyldimethylsilyl.

The term Troc means 2,2,2-trichloroethoxycarbonyl.

The term TCI means trichloroacetimidate.

The term Phth means phthaloyl.

The term TFA means trifluoroacetate.

The term TCA means trichloroacetate.

The term Cbz means benzyloxycarbonyl.

The term Bz means benzoyl.

The term Bn means benzyl.

The term TES means triethylsilyl.

The term TBDPS means tert-butyldiphenylsilyl.

The term MCA means monochloroacetate.

The term Lev means levulinoyl.

The term ADMB means 4-O-acetyl 2,2dimethylbutanoate.

The term Tr means triphenylmethyl.

The term DMT means dimethoxytrityl.

The term FMOC means 9-fluorenylmethyl carbonate.

The term Alloc means Allyloxycarbonyl.

The term Nap means napthyl.

The term SEt means thioethyl.

The term SPh means thiophenyl.

The term STol means thiotolyl.

The term SAdm means thioadamantyl.

### Synthetic oligosaccharides

The present invention provides compositions and methods for chemically synthesizing antigenic structures corresponding to a portion of the *P. aeruginosa* lipooligosaccharide (LOS), a major surface component of the outer membrane.

In oligosaccharides (**1a** and **1b**), oligosaccharides may include one or more monosaccharide units linked to one another through one or more α- and/or β-glycosidic bonds. Preferably, the oligosaccharides will include monosaccharides and glycosidic linkages naturally found in *P. aeruginosa* LOS structures, generally in 1-2 or 1-4 connectivities. The invention further contemplates other connectivities, such as 1-3 and 1-6, especially where the oligosaccharide design is extended beyond naturally *P. aeruginosa* LOS structures.

When either of R¹ or R² is an oligosaccharide, it may contain between 1 to about 6, preferably up to about 4, monosaccharide units. The invention contemplates inclusion of natural and modified monosaccharide units, such as glucose, fucosamine and rhamnose, especially where the oligosaccharide design is extended beyond naturally *P. aeruginosa* LOS structures.

In compounds (**1a** and **1b**), preferred saccharide substituents include α-Rha- (rhamnosyl), α-Glc(1-2)-α-Rha-, β-QuiNAc(1-3)-α-Rha-, β-FucNAc(1-3)-α-Rha-, α-Rha[2,3,4-OAc]-, β-QuiNAc(1-3)-α-Rha[2,4-OAc]-, and β-FucNAc(1-3)-α-Rha[2,4,-OAc]-.

In one aspect, the present invention provides oligosaccharides**1a:** 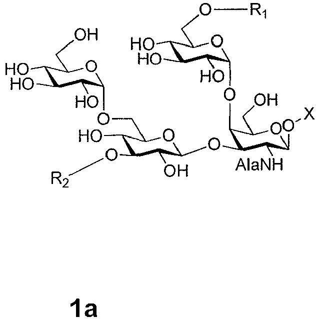 where each of R¹ and R² is independently H, a monosaccharide or an oligosaccharide, and X is H, a linker group or a protecting group. Preferably, R¹ and R² are selected from H, α-Rha- (rhamnosyl), α-Glc(1-2)-α-Rha-, β-QuiNAc(1-3)-α-Rha-, β-FucNAc(1-3)-α-Rha-, α-Rha[2,3,4-OAc]-, β-QuiNAc(1-3)-α-Rha[2,4-OAc]-, and β-FucNAc(1-3)-α-Rha[2,4,-OAc]-. More preferably, R¹ and R² are selected from the combinations in the table below:

| **R¹** | **R²** |
|---|---|
| α-Rha- | H |
| α-Glc(1-2)-α-Rha- | H |
| H | α-Rha- |
| H | β-QuiNAc(1-3)-α-Rha- |
| H | β-FucNAc(1-3)-α-Rha- |
| H | α-Rha[2,3,4-OAc]- |
| H | β-QuiNAc(1-3)-α-Rha[2,4-OAc]- |
| H | β-FucNAc(1-3)-α-Rha[2,4,-OAc]- |

In another aspect, the present invention provides oligosaccharides **1b**: 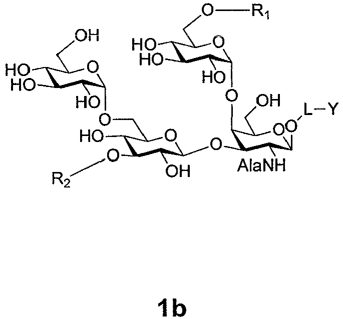 where each of R¹ and R² is independently H, a monosaccharide or an oligosaccharide, L is a linker group, and Y is a carrier. Preferably, R¹ and R² are selected from H, α-Rha- (rhamnosyl), α-Glc(1-2)-α-Rha-, β-QuiNAc(1-3)-α-Rha-, β-FucNAc(1-3)-α-Rha-, α-Rha[2,3,4-OAc]-, β-QuiNAc(1-3)-α-Rha[2,4-OAc]-, and β-FucNAc(1-3)-α-Rha[2,4,-OAc]-. More preferably, R¹ and R² are selected from the combinations in the table below:

| **R¹** | **R²** |
|---|---|
| α-Rha- | H |
| α-Glc(1-2)-α-Rha- | H |
| H | α-Rha- |
| H | β-QuiNAc(1-3)-α-Rha- |
| H | β-FucNAc(1-3)-α-Rha- |
| H | α-Rha[2,3,4-OAc]- |
| H | β-QuiNAc(1-3)-α-Rha[2,4-OAc]- |
| H | β-FucNAc(1-3)-α-Rha[2,4,-OAc]- |

In one embodiment, oligosaccharide (**1a**) has formula (**2a**):

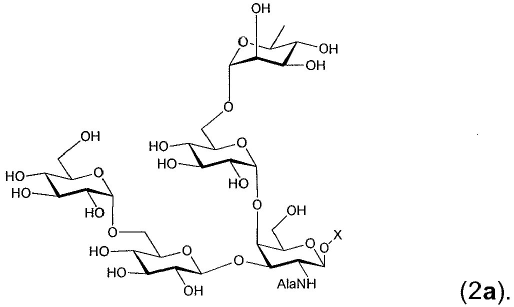

In another embodiment, oligosaccharide (**1b**) has formula (**2b**):

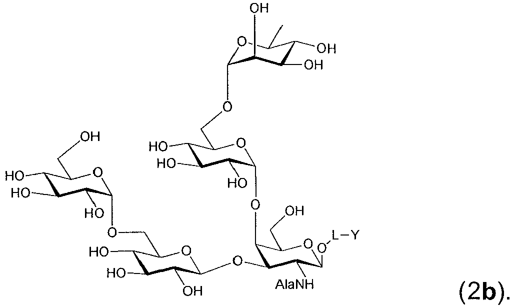

In a further embodiment, oligosaccharide (**1a**) has formula (**3a**):

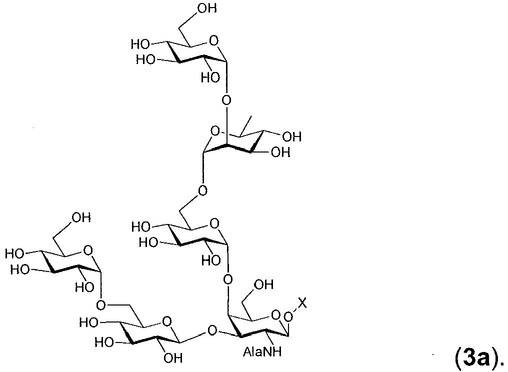

In a further embodiment, oligosaccharide (**1b**) has formula (**3b**):

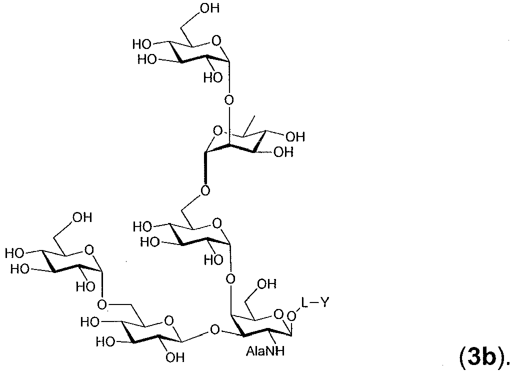

In a further embodiment, oligosaccharide (**1a**) has formula (**4a**):

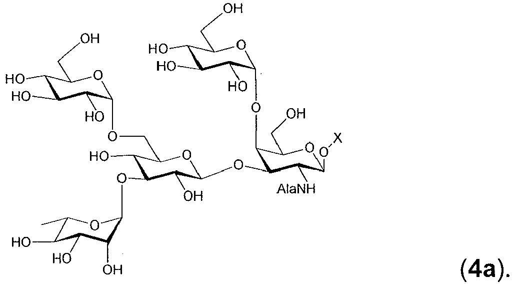

In a further embodiment, oligosaccharide (**1b**) has formula (**4b**):

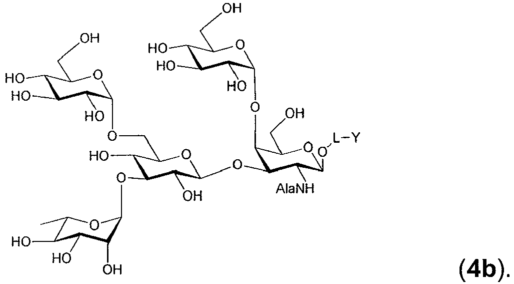

In a further embodiment, oligosaccharide (**1a**) has formula (**5a**):

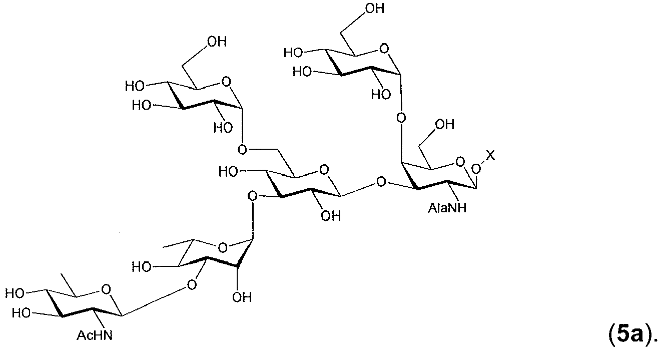

In a further embodiment, oligosaccharide (**1b**) has formula (**5b**):

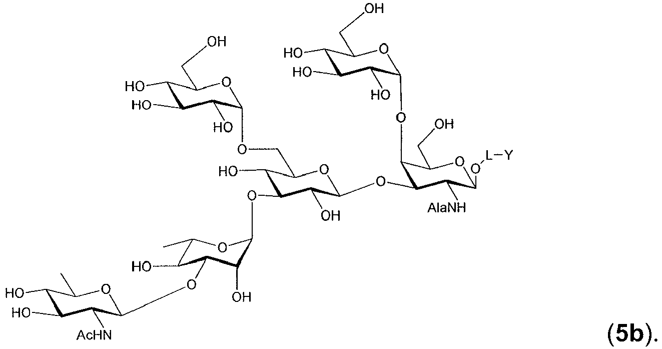

In a further embodiment, oligosaccharide (**1a**) has formula (**6a**):

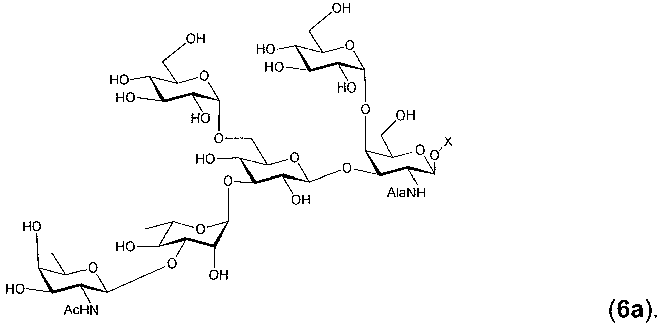

In a further embodiment, oligosaccharide (**1b**) has formula (**6b**):

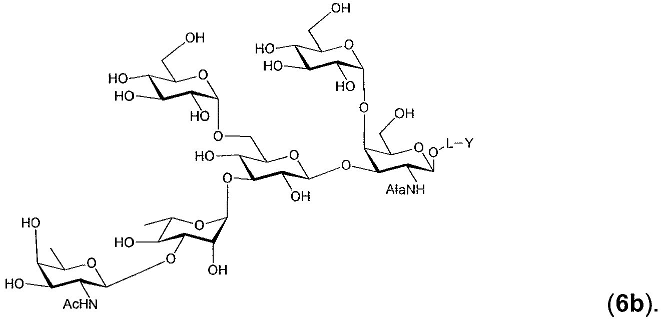

In a further embodiment, oligosaccharide (**1a**) has formula (**7a**):

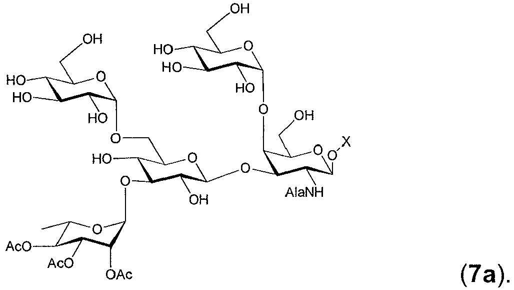

In a further embodiment, oligosaccharide (**1b**) has formula (**7b**):

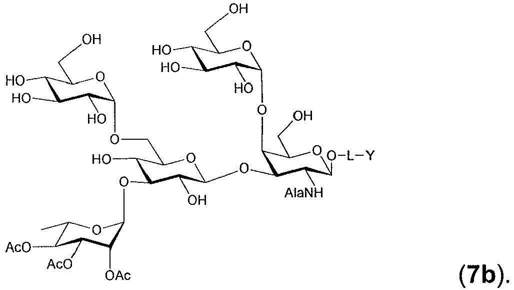

In a further embodiment, oligosaccharide (**1a**) has formula (**8a**):

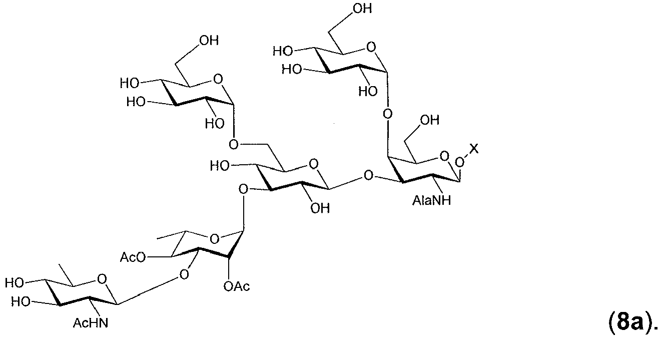

In a further embodiment, oligosaccharide (**1b**) has formula (**8b**):

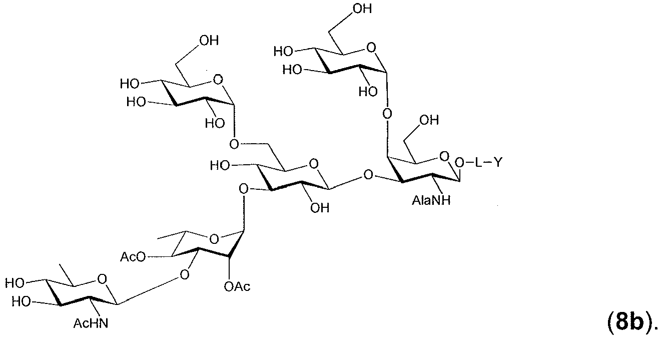

In a further embodiment, oligosaccharide (**1a**) has formula (**9a**):

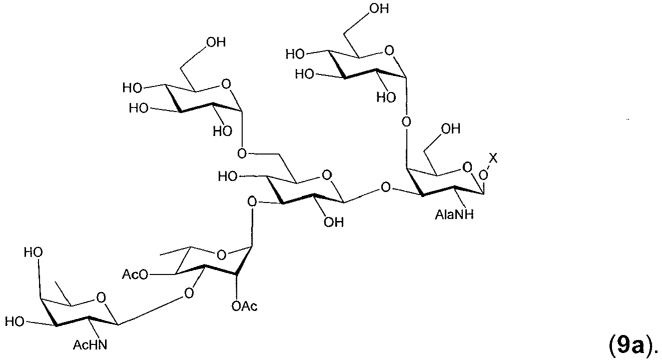

In a further embodiment, oligosaccharide (**1b**) has formula (**9b**):

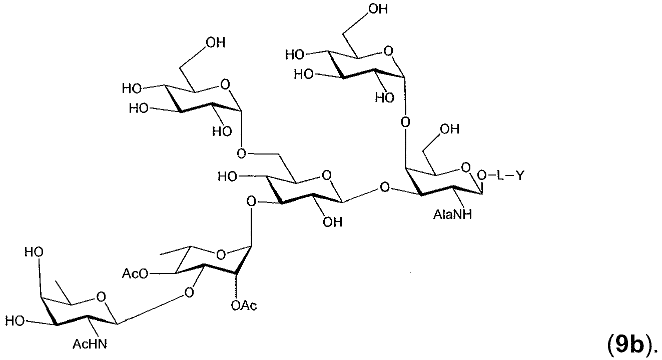

Table 1 lists preferred R¹ and R² combinations and Psuedomonas serotypes proposed to be covered by the permutations.

**Table 1.**

| **Glycoform from LOS outer core of Pseudomonas** | **R¹** | **R²** | **Potential IATS Serotype Coverage** |
|---|---|---|---|
| I | α-Rha- | H | All Serotypes |
| | α-Glc(1-2)-α-Rha- | H | All Serotypes |
| II | H | α-Rha- or | 3 |
| | H | β-QuiNAc(1-3)-α-Rha- or | 1,4,6,9,10,12,13,14,19 |
| | H | β-FucNAc(1-3)-α-Rha- or | 2,5,7,8,11,16,18,20 |

In another aspect, the present invention provides LOS antigens comprised of core oligosaccharide structures or motifs corresponding to one or more of the 20 major serotypes, members within a given serotype, and individual serotype subtypes as depicted in Table 1. In the following embodiments, the linker is exemplified as an alkylenethiol group having between 1 and 20 carbon atoms, preferably between 1 and 8. In any of these embodiments, the linker shown (i.e., the alkylenethiol group) could be replaced with any other suitable linker as described herein.

It should be recognized, that the present invention contemplates and provides sufficient guidance below for modifying any of the above-described thiol products with different linkers and/or spacers, and to make LOS structures directed to any of the oligosaccharides described above, including any subsequence combinations derived therefrom, or indeed, any *P. aeruginosa* LOS structure for that matter.

In a further aspect, the invention provides polyvalent LOS antigen combinations (and conjugates thereof) representing pluralities of any of the different oligosaccharides described in Table 1, for example, or multivalent combinations of one or more of the synthetic oligosaccharides 1a or 1b with one or more other protein antigens, carbohydrate O-antigens, and/or alginates

Suitable linkers comprise at one end a grouping able to enter into a covalent bonding with a reactive functional group of the carrier, e.g. an amino, thiol, or carboxyl group, and at the other end a grouping likewise able to enter into a covalent bonding with a hydroxyl group of an oligosaccharide according to the present invention. Between the two functional groups of the linker molecule there is a biocompatible bridging molecule of suitable length, e.g. substituted or unsubstituted heteroalkylene, arylalkylene, alkylene, alkenylene, or (oligo)alkylene glycol groups. Linkers preferably include substituted or unsubstituted alkylene or alkenylene groups containing 1-10 carbon atoms.

Linkers able to react with thiol groups on the carrier are, for example, maleimide and carboxyl groups; preferred groupings able to react with aldehyde or carboxyl groups are, for example, amino or thiol groups. Preferred covalent attachments between linkers and carriers include thioethers from reaction of a thiol with an α-halo carbonyl or α-halo nitrile, including reactions of thiols with maleimide; hydrazides from reaction of a hydrazide or hydrazine with an activated carbonyl group (e.g. activated NHS-ester or acid halide); triazoles from reaction of an azide with an alkyne (e.g. via "click chemistry"); and oximes from reaction of a hydroxylamine and an aldehyde or ketone as disclosed, for example, in Lees et al., Vaccine, 24:716, 2006. Further suitable linker molecules are known to skilled workers and commercially available or can be designed as required and depending on the functional groups present and can be prepared by known methods.

Suitable carriers are known in the art (See e.g., Remington's Pharmaceutical Sciences (18th ed., Mack Easton, PA (1990)) and may include, for example, proteins, peptides, lipids, polymers, dendrimers, virosomes, virus-like particles (VLPs), or combinations thereof, which by themselves may not display particular antigenic properties, but can support immunogenic reaction of a host to the oligosaccharides of the present invention (antigens) displayed at the surface of the carrier(s).

Preferably, the carrier is a protein *carrier,* including but not limited to, bacterial toxoids, toxins, exotoxins, and nontoxic derivatives thereof, such as tetanus toxoid, tetanus toxin Fragment C, diphtheria toxoid, CRM (a nontoxic diphtheria toxin mutant) such as CRM 197, cholera toxoid, *Staphylococcus aureus* exotoxins or toxoids, *Escherichia coli* heat labile enterotoxin, *Pseudomonas aeruginosa* exotoxin A, including recombinantly produced, genetically detoxified variants thereof; bacterial outer membrane proteins, such as *Neisseria meningitidis*serotype B outer membrane protein complex (OMPC), outer membrane class 3 porin (rPorB) and other porins; keyhole limpet hemocyanine (KLH), hepatitis B virus core protein, thyroglobulin, albumins, such as bovine serum albumin (BSA), human serum albumin (HSA), and ovalbumin; pneumococcal surface protein A (PspA), pneumococcal adhesin protein (PsaA); purified protein derivative of tuberculin (PPD); transferrin binding proteins, polyamino acids, such as poly(lysine:glutamic acid); peptidyiagonists of TLR-5 (e.g. flagellin of motile bacteria like Listeria); and derivatives and/or combinations of the above carriers. Preferred carriers for use in humans include tetanus toxoid, CRM 197, and OMPC.

Depending on the type of bonding between the linker and the carrier, and the structural nature of the carrier and oligosaccharide, a carrier may display on average, for example, 1 to 500, 1 to 100, 1 to 20, or 3 to 9 oligosaccharide units on its surface.

Methods for attaching an oligosaccharide to a carrier, such as a carrier protein are conventional, and a skilled practitioner can create conjugates in accordance with the present invention using conventional methods. Guidance is also available in various disclosures, including, for example, U.S. Pat. Nos. 4,356,170; 4,619,828; 5,153,312; 5,422,427; and 5,445,817; and in various print and online Pierce protein cross-linking guides and catalogs (Thermo Fisher, Rockford, IL).

In one embodiment, the carbohydrate antigens of the present invention are conjugated to CRM197, a commercially available protein carrier used in a number of FDA approved vaccines. CRM-conjugates have the advantage of being easier to synthesize, purify and characterize than other FDA approved carriers such as OMPC. Carbohydrate antigens may be conjugated to CRM via thiol-bromoacetyl conjugation chemistry. CRM activation may be achieved by reacting the lysine side chains with the NHS ester of bromoacetic acid using standard conditions as previously described in U.S. Pat. Appl. Publ. 2007-0134762. CRM may be functionalized with 10-20 bromoacetyl groups per protein (n=10-20) prior to conjugation. Conjugation may be performed at pH=9 to avoid aggregation of CRM. Careful monitoring of pH must be employed to ensure complete CRM reaction with NHS-bromoacetate while minimizing background hydrolysis of CRM. Activated CRM may be purified by size exclusion chromatography prior to conjugation. Antigen-CRM conjugates may be synthesized by reacting thiol-terminated carbohydrate antigens with bromoacetamide-activated CRM.

CRM conjugates may be purified via size exclusion chromatography to remove and recover any unreacted carbohydrate. MBTH (specific for GlcNAc residues) and Bradford assays may be used to determine carbohydrate:protein ratio and protein content, respectively, as previously described (Manzi et al., Curr. Prot. Mol. Biol., section 17.9.1 (Suppl. 32), 1995. In preferred embodiments, a minimum carbohydrate content of about 10% by weight for each conjugate may be generated. Typically, a conjugate may include about 3-20 antigens per protein carrier.

In another embodiment, carbohydrate antigens may be conjugated to one or more carriers suitable for development of diagnostic assays, including ELISAs and microarrays. Exemplary carriers for use in such assays include bovine serum albumin (BSA), keyhole limpet hemocyanine (KLH), biotin, a label, a glass slide or a gold surface. By way of example, synthetic carbohydrate antigens may be conjugated to BSA by a thiol-maleimide coupling procedure (FIG. 5B). Maleimide-BSA contains 15-20 maleimide groups per protein (n=15-20). Accordingly, oligosaccharide antigensmay be conjugated to maleimide functionalized BSA, whereby a 20-fold molar excess of the antigen is reacted with commercially available Imjectmaleimide BSA (Pierce) in maleimide conjugation buffer (Pierce). Conjugation may be performed at pH=7.2 to avoid hydrolysis of the maleimide group during conjugation.

BSA conjugates may be purified via size exclusion chromatography to remove and recover any unreacted carbohydrate. Characterization via the phenol-sulfuric acidand Bradford assays may be performed along with MALDI-MS to provide information on the carbohydrate content and valency of the conjugates. In preferred embodiments, conjugates will contain aminimum carbohydrate content of about 10% by weight per BSA conjugate and >8 antigen copies per conjugate.

### Methods for Synthesizing LOS-Oligosaccharide Structures

In a further aspect, the present invention provides a method for assembling synthetic homogenous LOS-oligosaccharide structures from *P. aeruginosa,* including those described above from monosaccharide and disaccharide building blocks.

### Scheme I shows P. aeruginosavaccine outer core retrosynthesis.

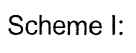

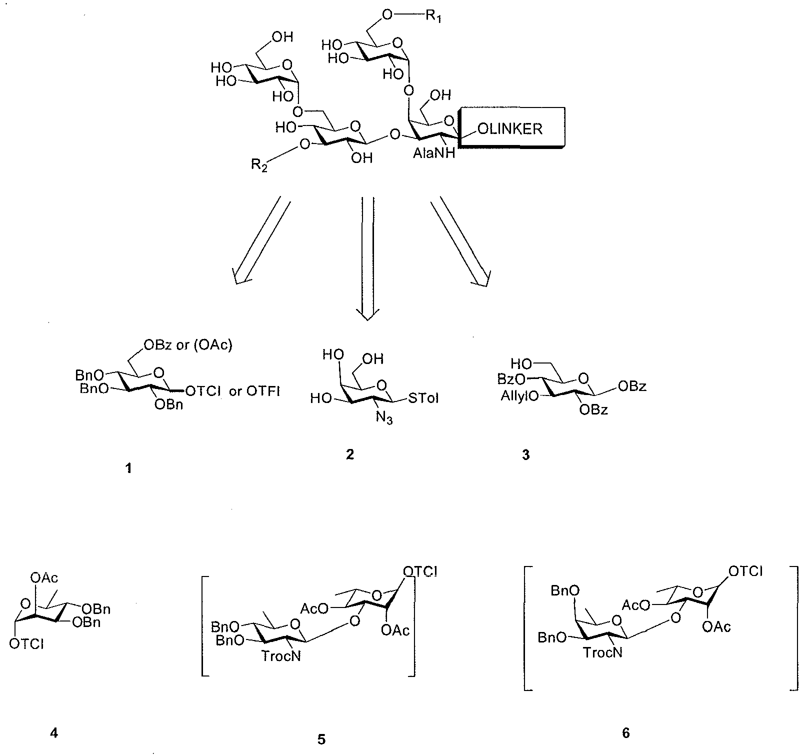

The outer core may be prepared according to procedures described in WO2012/082635 following the retrosynthesis in Scheme I.

α-Glucosyl donors may be prepared using the following scheme:

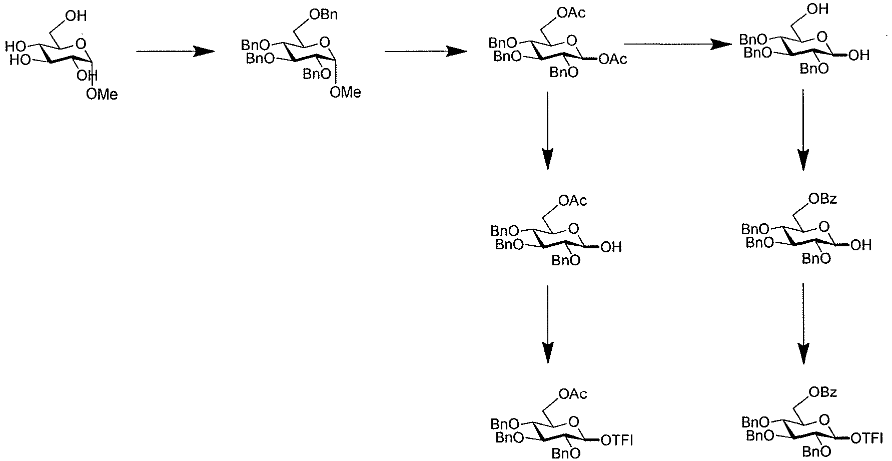

Galactosamine linker units suitable for preparing *P. aeruginosa* vaccine outer core synthesis may be prepared as follows:

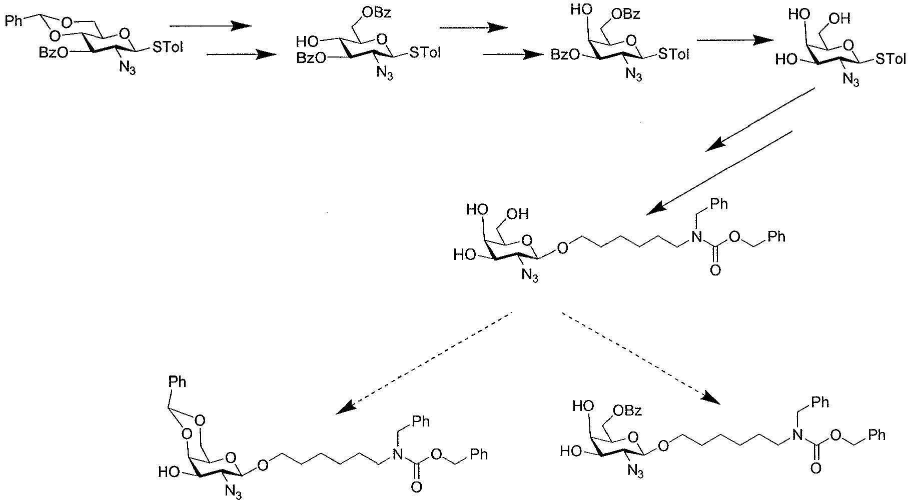

The hexyl(benzyl)carbamate compound may be prepared using procedures described by Whitfield D. M. et al (Collet. Czech. Chem. Commun. 58:159-17291993)). The 6-O and 4-O positions may be protected with benzylidine following Bedini E. et al. Carbohydrate Res. 349:24-32 (2012).

Branched glucose disaccharide units suitable for preparing *P. aeruginosa* vaccine outer core may be prepared as follows:

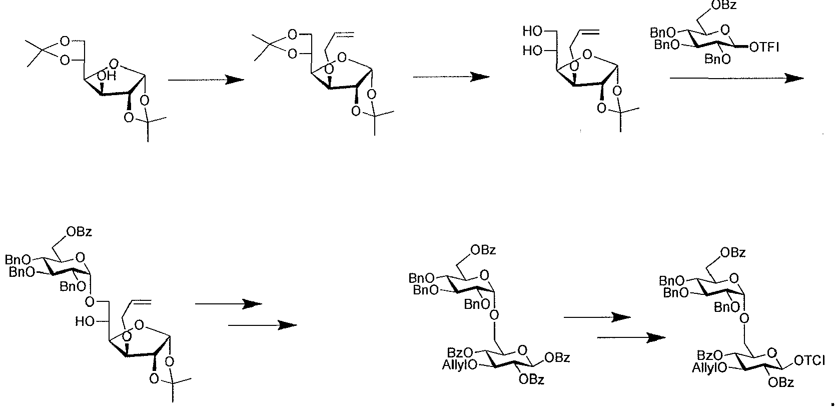

Core tetracaccharideassemplies suitable for preparing *P. aeruginosa* vaccine outer core may be prepared as following:

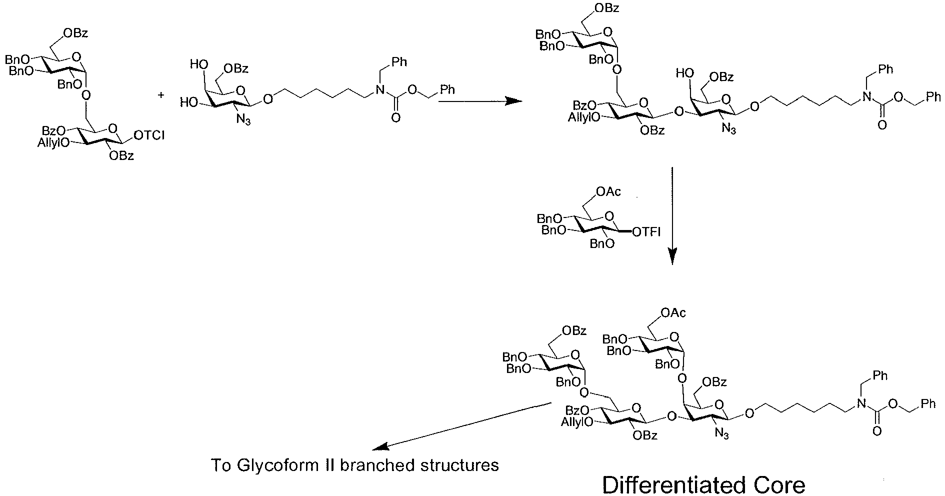

The selective coupling of GalN₃-3,4-diol with the disaccharide may be achieved by following procedures disclosed by Osswald et al. (Z. Naturforsch. 58b:764-774 (2003)) and Komarova B. S. et al. (Tetrahed. Lett. 47:3583-78 (2006)). Both references show the selectivity of the reaction in favor of 3-position over the 4-position. Komarova also describes a process for coupling the 4-position of the resulting trimer with OTFI-activated monosaccharide to provide a tetramer.

Compositions and methods for synthesis of the above described LOS-oligosaccharides and conjugates thereof, including others described in Table 1 are described in Examples 1 to 9 below. Protecting groups employed in the synthesis of LOS-oligosaccharides may include those customarily considered in sugar chemistry, for example those mentioned in "Protective Groups in Organic Synthesis", 3rd edition, T. W. Greene and P. G. M. Wuts (Ed.), John Wiley and Sons, New York, 1999.

### Immunogenic and Immunoprotective Compositions and Methods of their Use

In another aspect, the present invention provides immunogenic and immunoprotective compositions containing LOS oligosaccharides or LOS oligosaccharide-protein carrier conjugates for inducing an immune response to LOS antigens. The immunogenic compositions may include one or more adjuvants, as well as pharmaceutically acceptable vehicles suitable for administration to an animal or individual. An immunogenic or immunoprotective composition will include a "sufficient amount" or "an immunologically effective amount" of a oligosaccharide-protein carrier conjugate according to the present invention, as well as any of the above mentioned components, for purposes of generating an immune response or providing protective immunity, as further defined herein.

In one embodiment, the invention provides an immunogenic composition comprising one or more LOS oligosaccharide(s) **1a** or LOS oligosaccharide-protein carrier conjugate(s) **1b** suitable for inducing an immune response against *P. aeruginosa.*

In another embodiment, the invention provides a pharmaceutical composition comprising a LOS oligosaccharide(s) **1a** or LOS oligosaccharide-protein carrier conjugate **1b** formulated as a vaccine for protection against *P. aeruginosa* infections.

In another embodiment, the invention provides a pharmaceutical composition comprising an oligosaccharide-protein carrier conjugate **1b** formulated as a vaccine for protecting against one or more *P. aeruginosa* serotypes as described herein.

In a further embodiment, the invention provides a pharmaceutical composition comprising an antibody and a physiologically acceptable vehicle for use in a method for providing passive immunity or treatment against one or more *P. aeruginosa* serotypes. More particularly, the invention provides an antibody preparation against one or more LOS-oligosaccharide conjugate **1b** compositions in accordance with the present invention. The antibody preparation may include any member from the group consisting of polyclonal antibody, monoclonal antibody, mouse monoclonal IgG antibody, humanized antibody, chimeric antibody, fragment thereof, or combination thereof. The invention further contemplates a hybridoma cell producing a monoclonal antibody directed against any of the LOS-oligosaccharide described herein.

Administration of oligosaccharides or oligosaccharide-protein carrier conjugates or antibodies thereto may be carried out by any suitable means, including by parenteral administration (e.g., intravenously, subcutaneously, intradermally, or intramuscularly); by topical administration, of for example, antibodies to an airway surface; by oral administration; by in ovo injection in birds, for example, and the like.

In specific embodiments, each immunogenic or immunoprotective composition includes one or more oligosaccharide(s) according to Formula **1a** or **1b** or conjugates thereof in a pharmaceutically acceptable vehicle or diluent forming a substantially aqueous mixture. In preferred embodiments, the immunogenic or immunoprotective compositions includes one or more oligosaccharide-protein carrier conjugates(s) in conjunction with one or more pharmaceutically acceptable adjuvant(s), vehicles and/or protein carriers suitable for administration to an animal or individual.

### Adjuvants

An oligosaccharide-protein carrier conjugate composition may further include one or more immunologic adjuvant(s). An immunologic adjuvant is a compound that, when combined with an antigen, increases the immune response to the antigen as compared to the response induced by the antigen alone so that less antigen can be used to achieve a similar response. For example, an adjuvant may augment humoral immune responses, cell-mediated immune responses, or both.

Those of skill in the art will appreciate that the terms "adjuvant,"and "carrier," can overlap to a significant extent. For example, a substance which acts as an "adjuvant" may also be a "carrier," and certain other substances normally thought of as "carriers," for example, may also function as an "adjuvant." Accordingly, a substance which may increase the immunogenicity of the synthetic oligosaccharide or carrier associated therewith is a potential adjuvant. As used herein, a carrier is generally used in the context of a more directed site-specific conjugation to an oligosaccharide of the present invention, whereby an adjuvant is generally used in a less specific or more generalized structural association therewith.

Exemplary adjuvants and/or adjuvant combinations may be selected from the group consisting of mineral salts, including aluminum salts, such as aluminum phosphate and aluminum hydroxide (alum) (e.g., Alhydrogel™, Superfos, Denmark) and calcium phosphate; RIBI, which contains three components extracted from bacteria, monophosphoryl lipid A, trehalosedimycolate, and cell wall skeleton (MPL+TDM+CWS) in a 2% squalene/Tween 80 emulsion, whereby any of the 3 components MPL, TDM or CWS may also be used alone or combined 2 by 2; toll-like receptor (TLR) agonists, including, for example, agonists of TLR-1 (e.g. tri-acyl lipopeptides); agonists of TLR-2 [e.g. peptidoglycan of gram-positive bacteria like streptococci and staphylococci; lipoteichoic acid]; agonists of TLR-3 (e.g. double-stranded RNA and their analogs such as poly 1:C); agonists of TLR-4 (e.g. lipopolysaccharide (endotoxin) of gram-negative bacteria like Salmonella and E. coli); agonists of TLR-5 (e.g. flagellin of motile bacteria like Listeria); agonists of TLR-6 (e.g. with TLR-2 peptidoglycan and certain lipids (diacyllipopeptides)); agonists of TLR-7 (e.g. single-stranded RNA (ssRNA) genomes of such viruses as influenza, measles, and mumps; and small synthetic guanosine-base antiviral molecules like loxoribine and ssRNA and their analogs); agonists of TLR-8 (e.g. binds ssRNA); agonists of TLR-9 (e.g. unmethylatedCpG of the DNA of the pathogen and their analogs; agonists of TLR-10 (function not defined) and TLR-11-(e.g. binds proteins expressed by several infectious protozoans (Apicomplexa), specific toll-like receptor agonists include monophosphoryl lipid A (MPL®), 3 De-O-acylatedmonophosphoryl lipid A (3 D-MPL), OM-174 (E. coli lipid A derivative); OM triacyl lipid A derivative, and other MPL- or lipid A-based formulations and combinations thereof, including MPL®-SE, RC-529 (Dynavax Technologies), AS01 (liposomes+MPL+QS21), AS02 (oil-in-water PL + QS-21), and AS04 (Alum + MPL)(GlaxoSmith Kline, Pa.), CpG-oligodeoxynucleotides (ODNs) containing immunostimulatoryCpG motifs, double-stranded RNA, polyinosinic:polycytidylic acid (poly I:C), and other oligonucleotides or polynucleotides optionally encapsulated in liposomes; oil-in-water emulsions, including AS03 (GlaxoSmith Kline, Pa.), MF-59 (microfluidized detergent stabilized squalene oil-in-water emulsion; Novartis), and Montanide ISA-51 VG (stabilized water-in-oil emulsion) and Montanide ISA-720 (stabilized water/squalene; Seppic Pharmaceuticals, Fairfield, NJ); cholera toxin B subunit; saponins, such as Quil A or QS21, an HPLC purified non-toxic fraction derived from the bark of QuillajaSaponaria Molina (STIMULON™ (Antigenics, Inc., Lexington, Mass.) and saponin-based adjuvants, including immunostimulating complexes (ISCOMs; structured complex of saponins and lipids) and other ISCOM-based adjuvants, such as ISCOMATRIX™ and AblSCO®-100 and -300 series adjuvants (Isconova AB, Uppsala, Sweden); QS21 and 3 D-MPL together with an oil in water emulsion as disclosed in U.S. Pat. Appl. No. 2006/0073171; stearyl tyrosine (ST) and amide analogs thereof; virus-like particles (VLPs) and reconstituted influenza virosomes (IRIVs);complete Freund's adjuvant (CFA); incomplete Freund's adjuvant (IFA); E. coli heat-labile enterotoxin (LT); immune-adjuvants, including cytokines, such as IL-2, IL-12, GM-CSF, Flt3, accessory molecules, such as B7.1, and mast cell (MC) activators, such as mast cell activator compound 48/80 (C48/80);water-insoluble inorganic salts; liposomes, including those made from DNPC/Chol and DC Chol; micelles; squalene; squalane; muramyl dipeptides, such as N-acetyl-muramyl-L-threonyl-D-isoglutamine (thr-MDP) as found in U.S. Pat. No. 4,606,918, N-acetyl-normuramyl-L-alanyl-D-isoglutamine (nor-MDP), and N-acetylmuramyl-L-alanyl-D-isoglutaminyl-L-alanine-2-(1'2'-dipalmitoyl-n-glycero-3-hydroxyphosphoryl; SAF-1 (Syntex); AS05 (GlaxoSmith Kline, Pa.); and combinations thereof.

In preferred embodiments, adjuvant potency may be enhanced by combining multiple adjuvants as described above, including combining various delivery systems with immunopotentiating substances to form multi-component adjuvants with the potential to act synergistically to enhance antigen-specific immune responses *in vivo.* Exemplary immunopotentiating substances include the above-described adjuvants, including, for example, MPL and synthetic derivatives, MDP and derivatives, oligonucleotides (CpGetc), ds RNAs, alternative pathogen-associated molecular patterns (PAMPs)(*E*. *coli* heat labile enterotoxin; flagellin, saponins (QS-21 etc), small molecule immune potentiators (SMIPs, e.g., resiquimod [R848]), cytokines, and chemokines.

### Pharmaceutically-acceptable delivery vehicles

Pharmaceutically-acceptable delivery vehicles, including those described above may be employed to enhance the delivery and/or control the duration of action. Control release preparations may be achieved through the use of polymers to complex or absorb the oligosaccharides, oligosaccharide conjugates, and/or adjuvants. Controlled delivery may be effected by selecting appropriate macromolecules (for example polyesters, polyamino acids, polyvinyl, pyrrolidone, ethylenevinylacetate, methylcellulose, carboxymethylcellulose, or protamine sulfate) and the concentration of macromolecules as well as the method of incorporation in order to control release. Another possible method to control the duration of action by controlled release preparations is to incorporate the compounds of the present invention into particles of a polymeric material such as polyesters, polyamino acids, hydrogels, poly(lactic acid) or ethylene vinylacetate copolymers. Alternatively, instead of incorporating these agents into polymeric particles, it is possible to entrap these materials in microcapsules prepared, for example, interfacial polymerization, for example, hydroxymethylcellulose or gelatin-microcapsules and poly(methylmethacylate)-microcapsules, respectively, or in colloidal drug delivery systems, for example, liposomes, albumin microspheres, microemulsions, nanoparticles, and nanocapsules or in macroemulsions. Such techniques are disclosed in Remington's Pharmaceutical Sciences, supra.

The oligosaccharide compositions of the present invention, including oligosaccharide-protein carrier conjugate compositions, can be formulated according to known methods to prepare pharmaceutically useful compositions, whereby these materials, or their functional derivatives, are combined in admixture with a pharmaceutically acceptable vehicle (or diluents). Suitable vehicles and their formulation, inclusive of other human proteins, e.g., human serum albumin, are described, for example, in Remington's Pharmaceutical Sciences, supra. In order to form a pharmaceutically acceptable composition suitable for effective administration, such compositions will contain an effective amount of the above-described compounds together with a suitable amount of protein carrier and/or vehicle.

Typically, the immunogenic or immunoprotective compositions may be prepared as injectables, either as liquid solutions or suspensions; solid forms suitable for solution in, or suspension in, liquid vehicles prior to injection. An aqueous composition for parenteral administration, for example, may include a solution of the immunogenic component(s) dissolved or suspended in a pharmaceutically acceptable vehicle or diluent, preferably a primarily aqueous vehicle. Pharmaceutically acceptable vehicles or diluents may include water, saline, including neutral saline solutions buffered with phosphate, Tris, glycerol, ethanol, and the like. An aqueous composition may be formulated as a sterile, pyrogen-free buffered saline or phosphate-containing solution, which may include a preservative or may be preservative free. Suitable preservatives include benzyl alcohol, parabens, thimerosal, chlorobutanol, and benzalkonium chloride, for example. Aqueous solutions are preferably approximately isotonic, and its tonicity may be adjusted with agents such as sodium tartrate, sodium chloride, propylene glycol, and sodium phosphate. Additionally, auxiliary substances required to approximate physiological conditions, including pH adjusting and buffering agents, tonicity adjusting agents, wetting or emulsifying agents, pH buffering substances, and the like, including sodium acetate, sodium lactate, sodium chloride, potassium chloride, calcium chloride, sorbitanmonolaurate, triethanolamineoleate, etc. may be included with the vehicles described herein.

These compositions may be sterilized by conventional sterilization techniques, or may be sterile filtered. The resulting aqueous solutions may be packaged for use as is, or lyophilized, the lyophilized preparation being combined with a sterile solution prior to administration. The preparation of such pharmaceutical compositions is within the ordinary skill in the art, and may be guided by standard reference books such as Remington's Pharmaceutical Science, supra.

Compositions may be formulated in a solid or liquid form for oral delivery. For solid compositions, nontoxic and/or pharmaceutically acceptable solid protein carriers may include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition may be formed by incorporating any of the normally employed excipients, including those protein carriers previously listed, and a unit dosage of an active ingredient, that is, one or more compounds of the invention, whether conjugated to a protein carrier or not.

Topical application of antibodies to an airway surface, for example, can be carried out by intranasal administration (e.g., by use of dropper, swab, or inhaler which deposits a pharmaceutical formulation intranasally). Topical application of the antibodies to an airway surface can also be carried out by inhalation administration, such as by creating respirable particles of a pharmaceutical formulation (including both solid particles and liquid particles) containing the antibodies as an aerosol suspension, and then causing the subject to inhale the respirable particles. Methods and apparatuses for administering respirable particles of pharmaceutical formulations are well known, and any conventional technique can be employed. Oral administration may be in the form of an ingestable liquid or solid formulation.

Further, compositions may be formulated in an aerosol for nasal administration. For aerosol administration, the immunogenic compounds are preferably supplied in finely divided form along with one or more surfactant(s) and/or propellant(s). The surfactant will be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. The surfactant may constitute 0.1%-20% by weight of the composition, preferably 0.25-5%. The balance of the composition is ordinarily propellant. A protein carrier can also be included, as desired, as with, e.g., lecithin for intranasal delivery.

The concentration of the immunogenic oligosaccharides of the invention in the pharmaceutical formulations can vary widely, i.e., from less than about 0.1%, usually at or at least about 0.1% to as much as 20% to 50% or more by weight, and will be selected primarily by fluid volumes, viscosities, etc., and in accordance with the particular mode of administration selected. A human unit dose form of the compounds and composition is typically included in a pharmaceutical composition that comprises a human unit dose of an acceptable protein carrier, preferably an aqueous protein carrier, and is administered in a volume of fluid that is known by those of skill in the art to be used for administration of such compositions to humans, and is adjusted according to commonly understood principles for a particular subject to be treated. Thus in one embodiment, the invention provides a unit dosage of the vaccine components of the invention in a suitable amount of an aqueous solution, such as 0.1-3 ml, preferably 0.2-2 mL.

### Methods of treatment

The immunogenic and immunoprotective compositions of the present invention may be administered to any animal species at risk for developing an infection by *P. aeruginosa.*

The treatment may be given in a single dose schedule, or preferably a multiple dose schedule in which a primary course of treatment may be with 1-10 separate doses, followed by other doses given at subsequent time intervals required to maintain and or reinforce the response, for example, at 1-4 months for a second dose, and if needed, a subsequent dose(s) after several months.

Examples of suitable treatment schedules include: (i) 0, 1 month and 6 months, (ii) 0, 7 days and 1 month, (iii) 0 and 1 month, (iv) 0 and 6 months, or other schedules sufficient to elicit the desired responses expected to reduce disease symptoms, or reduce severity of disease.

The amounts effective for inducing an immune response or providing protective immunity will depend on a variety of factors, including the oligosaccharide composition, conjugation to a protein carrier, inclusion and nature of adjuvant(s), the manner of administration, the weight and general state of health of the patient, and the judgment of the prescribing physician. By way of example, the amounts may generally range for the initial immunization (that is for a prophylactic administration) from about 1.0 µg to about 5,000 µg of carbohydrate antigen for a 70 kg patient, (e.g., 1.0 µg, 2.0 µg, 2.5 µg, 3.0 µg, 3.5 µg, 4.0 µg, 4.5 µg, 5.0 µg, 7.5 µg, 10 µg, 12.5 µg, 15 µg, 17.5 µg, 20 µg, 25 µg, 30 µg, 35 µg, 40 µg, 45 µg, 50 µg, 75 µg, 100 µg, 250 µg, 500 µg, 750 µg, 1,000 µg, 1,500 µg, 2,000 µg, 2,500 µg, 3,000 µg, 3,500 µg, 4,000 µg, 4,500 µg or 5,000 µg). The actual dose administered to a subject is often determined according to an appropriate amount per kg of the subject's body weight. For example, an effective amount may be about 0.1 µg to 5 µg/kg body weight.

A primary dose may optionally be followed by boosting dosages of from about 1.0 to about 1,000 of carbohydrate antigen (e.g., 1.0 µg, 2.0 µg, 2.5 µg, 3.0 µg, 3.5 µg, 4.0 µg, 4.5 µg, 5.0 µg, 7.5 µg, 10 µg, 12.5 µg, 15 µg, 17.5 µg, 20 µg, 25 µg, 30 µg, 35 µg, 40 µg, 45 µg, 50 µg, 75 µg, 100 µg, 250 µg, 500 µg, 750 µg, 1,000 µg, 1,500 µg, 2,000 µg, 2,500 µg, 3,000 µg, 3,500 µg, 4,000 µg, 4,500 µg or 5,000 µg) pursuant to a boosting regimen over weeks to months depending upon the patient's response and condition by measuring specific T cell activity in the patient's blood.

The present invention contemplates the use of single- and multi-valent glycoconjugate vaccines comprising any of the synthetic oligosaccharides described herein. The identification of a single oligosaccharide antigen eliciting a cross-reactive immune response can facilitate development of a single-antigen vaccine candidate active against all common *P. aeruginosa* bacterial serotypes and/or strains.

The present invention further contemplates multi-antigen glycoconjugate vaccines comprising a plurality of any of the synthetic oligosaccharides described herein so as to provide protection against a single serotype or serotype subtype of *P. aeruginosa* or against a plurality of serotypes or serotype subtypes of *P. aeruginosa.* Thus, in one embodiment, for example, the invention provides a composition containing two, three, four or more different oligosaccharide antigens according to Formula **1b.**

The immunogenic compositions comprising a compound of the invention may be suitable for use in adult humans or in children, including young children or others at risk for contracting an infection caused by a LOS-expressing bacterial species. Optionally such a composition may be administered in combination with other pharmaceutically active substances, and frequently it will be administered in combination with other vaccines as part of a childhood vaccination program.

Compositions for administration may beneficially include multiple oligosaccharide- or oligosaccharide conjugates that elicit an immune response to a plurality of different epitopes so as to provide increased protection against a single strain or serotype of *P. aeruginosa* or against a plurality of strains or serotypes of *P. aeruginosa.* Moreover, compositions may be administered whereby a prime immunization with one or multiple antigen conjugates is followed by boosting events with one or more cross-reactive core conjugates according to the present invention.

### Antibody compositions

In another embodiment, the invention provides diagnostic antibodies, as well as pharmaceutical compositions comprising one or more anti-LOS antibody(ies) or a functional fragment(s) thereof, and a physiologically acceptable vehicle. Methods for generating these antibodies are further described below.

Pharmaceutical antibody compositions may be used in a method for providing passive immunity against *P. aeruginosa* infections. A pharmaceutical antibody composition may be administered to an animal subject, preferably a human, in an amount sufficient to prevent or attenuate the severity, extent of duration of the infection by one or more strains or serotypes of *P. aeruginosa.*

The administration of one or more antibodies may be either prophylactic (prior to anticipated exposure to a bacterial infection) or therapeutic (after the initiation of the infection, at or shortly after the onset of the symptoms).The dosage of the one or more antibodies will vary depending upon factors as the subject's age, weight and species. In general, the dosage of the antibody may be in a range from about 1-10 mg/kg body weight. In a preferred embodiment, the antibody is a humanized antibody of the IgG or the IgA class. The route of administration of the one or more antibodies may be oral or systemic, for example, subcutaneous, intramuscular or intravenous.

The use of antibodies as diagnostic agents is further described below and in U.S. Pat. No. 7,595,307 and U.S. Pat. Appl. Publ. No. 2009/0155299.

The present invention also provides one or more kits useful for diagnosing, treating, and/or preventing a *P. Aeruginosa* infection. For example, the kits may include one or more containers holding the diagnostic or pharmaceutical compositions of the invention. The kits may also include other container(s) containing, for example, one or more solutions necessary or convenient for the particular diagnostic or pharmaceutical use. The container means can be made of glass, plastic or foil and can be a vial, bottle, pouch, tube, bag, etc. The kit may also contain written information, such as procedures for carrying out the present invention or analytical information, such as the amount of reagent contained in the container(s).

### Generation of antibodies and their use in assay development

In a further aspect, the present invention provides compositions and methods for inducing production of antibodies for use in assay development, including their use as detection agents and serum screening tools.

Antisera to LOS-conjugates may be generated in New Zealand white rabbits by 3-4 subcutaneous injections over 13 weeks. A pre-immune bleed may generate about 5 mL of baseline serum from each rabbit. A prime injection (10 µg antigen equivalent) may be administered as an emulsion in complete Freund's adjuvant (CFA). Subsequent injections (5 µg antigen equivalent) may be given at three week intervals in incomplete Freund's adjuvant (IFA). Rabbits may be bled every two weeks commencing one week after the third immunization. Approximately 25 - 30 mL of serum per rabbit may be generated from each bleeding event and frozen at -80°C. Serum may be analyzed by ELISA against the corresponding LOS-conjugate as described below. In addition, antisera from later bleeds may be affinity purified as further described below.

The oligosaccharides and antibodies of the present invention can be used as diagnostic reagents for detecting *P. aeruginosa* LOS antigens or antibodies thereagainst, which are present in biological samples. The detection reagents may be used in a variety of immunodiagnostic techniques, known to those of skill in the art, including ELISA- and microarray-related technologies. In addition, these reagents may be used to evaluate antibody responses, including serum antibody levels, to immunogenic oligosaccharide conjugates. The assay methodologies of the invention typically involve the use of labels such as fluorescent, chemiluminescent, radioactive, enzymatic labels or dye molecules, and/or secondary immunologic reagents for direct or indirect detection of a complex between an antigen or antibody in a biological sample and a corresponding antibody or antigen bound to a solid support.

Such assays typically involve separation of unbound antibody in a liquid phase from a solid phase support to which antibody-antigen complexes are bound. Solid supports which can be used in the practice of the invention include substrates such as nitrocellulose (e.g., in membrane or microtiter well form); polyvinylchloride (e.g., sheets or microtiter wells); polystyrene latex (e.g., beads or microtiter plates); polyvinylidine fluoride; diazotized paper; nylon membranes; activated beads, magnetically responsive beads, and the like.

Typically, a solid support is first reacted with a first binding component (e.g., an antigen or antibody in accordance with the present invention) under suitable binding conditions such that the first binding component is sufficiently immobilized to the support. In some cases, mobilization to the support can be enhanced by first coupling the antibody or oligosaccharide to a protein with better binding properties, or that provides for immobilization of the antibody or antigen on the support without significant loss of antibody binding activity or specificity. Suitable coupling proteins include, but are not limited to, macromolecules such as serum albumins including bovine serum albumin (BSA), keyhole limpet hemocyanin (KLH), immunoglobulin molecules, thyroglobulin, ovalbumin, and other proteins well known to those skilled in the art. Other molecules that can be used to bind antibodies the support include polysaccharides, polylactic acids, polyglycolic acids, polymeric amino acids, amino acid copolymers, and the like. Such molecules and methods of coupling these molecules are well known to those of ordinary skill in the art and are described in, e.g., U.S. Pat. No. 7,595,307 and U.S. Pat. Appl. No. US 2009/0155299.

### EXAMPLES

It is understood that the examples and embodiments described herein are for illustrative purposes only and that various modifications or changes in light thereof will be suggested to persons skilled in the art and are to be included within the spirit and purview of this application and scope of the appended claims.

### Experimental Details:

The following schemes detail experimental protocolos for the production of key building blocks useful for the assembly of P. *aeruginosa* LOS antigens as described above. In each of **Scheme 1-5,** solid arrows indicate chemistry that was performed and dashed arrow represent chemistry that is prophetic.

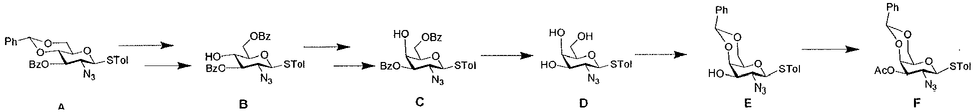

### Scheme 1. Synthesis of Glucosamine Building Block F

### Synthesis of Intermediate E:

To a solution of the known triol **D** (40 mmol), prepared according to Emmadi, M., Kulkarni, S.S. J. Org. Chem. 2011, 76, 4703, in anhydrous DMF (100 mL) were added benzaldehyde dimethylacetal (7.22 mL, 48 mmol) and camphor sulfonic acid (2 g). The reaction mixture was warmed to 50°C under vacuum on a rotary evaporator. After 1h, the reaction mixture was quenched with triethylamine (6 mL) and concentrated *in vacuo.* The crude product **E** was used in the next step without purification.

### Synthesis of Intermediate F:

Crude intermediate **E** (∼40 mmol) was dissolved in pyridine (80 mL). The reaction mixture was cooled to 0°C and acetic anhydride (40 mL) was added. The reaction mixture stirred at room temperature for 12 h, then concentrated to a thick slurry. EtOAc (200 mL) were added and the suspension was partitioned with water (200 mL). The organics were collected, washed with NaHCO₃ and brine. The crude product was dried over Na₂SO₄, filtered and concentrated. Purification via silica gel chromatography (EtOAc, Heptane eluent) afforded intermediate **F** (11 g).

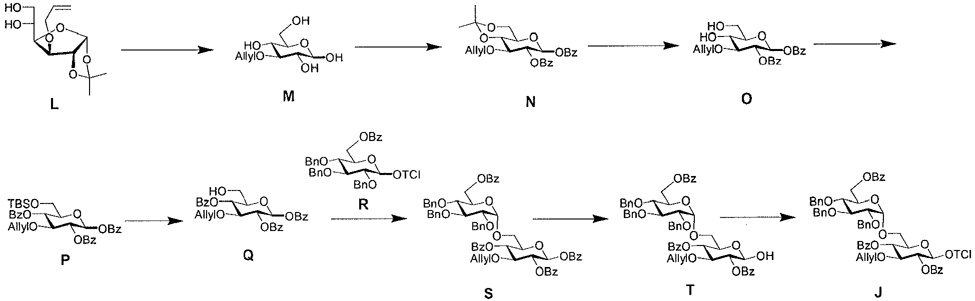

### Scheme 2. Synthesis of Glucose Disaccharide Building Block J

### Synthesis of Intermediate N:

Known compound **M** (Peri, F. et al J. Carbohydr. Chem. 2003, 22, 57) (275 mmol) was co-evaporated with DMF (3 x 100 mL) and dissolved in CH₂Cl₂ (100 mL). 2-methoxypropene (52 mL, 550 mmol) was added and the reaction mixture was cooled to 0°C for 30 min. p-Toluenesulfonic acid (120 mg) was added and the reaction mixture was allowed to warm to room temperature over 12 h. The resulting mixture was neutralized with Na₂CO₃ (20 g) and stirred for an additional 1 h. The suspension was filtered through celite, rinsed with EtOAc and washed with water and brine. The crude product was dissolved in pyridine (200 mL), cooled to 0°C and benzoyl chloride (80 mL) were added dropwise. The reaction mixture was warmed to room temperature and stirred for 12 h. After cooling to 0°C, methanol (40 mL) were added and stirred for 30 min. The reaction mixture was concentrated to a viscous white solid. The suspension was dissolved in EtOAc (500 mL) and washed with washed with water and brine. The crude product was dried over Na₂SO₄, filtered and concentrated. Product **N** was used in the next step without purification.

### Synthesis of Intermediate O:

Intermediate **N** (∼275 mmol) was suspended in HOAc:H₂O (500 mL, 4:1 v:v) and stirred at room temperature for 18 h. The reaction mixture was concentrated *in vacuo* to a thick syrup. The suspension was co-evaporated with toluene (3x100 mL) and purified on silica gel. Intermediate **O** (49.8 g) was isolated.

### Synthesis of Intermediate P:

Intermediate **O** (116 mmol) was co-evaporated with pyridine (3 x 100 mL) and dissolved in pyridine (300 mL). The reaction mixture was cooled to 0°C for 30 min. TBSCI (139 mmol, dissolved in 100 mL CH₂Cl₂) were added dropwise and the reaction mixture was allowed to warm to room temperature over 12 h. The resulting mixture was cooled to 0°C for 30 min. Benzoyl chloride (162 mmol) were added and the reaction mixture was warmed to room temperature over 18 h. Methanol (40 mL) were added and stirred for 30 min. The reaction mixture was concentrated to a viscous white solid. The suspension was dissolved in EtOAc (500 mL) and washed with washed with 1M HCl, water and brine. The mixture was was dried over Na₂SO₄, filtered and concentrated. Product **P** was used in the next step without purification.

### Synthesis of Intermediate Q:

Intermediate **P** (∼116 mmol) was suspended in CH₂Cl₂ (200 mL) and treated with TFA:H₂O (22 mL, 10:1 v:v) and stirred at room temperature for 1 h. The reaction mixture was concentrated *in vacuo* to a thick syrup. The suspension was co-evaporated with toluene (3x100 mL) and purified on silica gel. Intermediate **Q** (82 mmol) was isolated as a mixture of α- and β- isomers.

### Synthesis of Disaccharide S:

Known glycosyl trichloroacetimidate **R** (21.7 mmol) and glycosyl acceptor **Q** (26.3 mmol) were combined, co-evaporated with toluene (3 x 20 mL) and dried *in vacuo* for 1h. The resulting mixture was dissolved in dry CH₂Cl₂ (150 mL) an dry diethyl ether (100 mL), purged under nitrogen and cooled to -20°C. A solution of trimethylsilyl trifluoromethanesulfonate (TMSOTf, 1.0 M in CH₂Cl₂, 2.19 mL, 2.19 mmol) was added dropwise over 10 minutes and the reaction stirred for an additional 30 minutes at -10°C. The reaction mixture was quenched with triethylamine (2 mL), diluted with CH₂Cl₂ (50 mL) and washed with saturated aqueous NaHCO₃ and brine. The organic solution was dried over Na₂SO₄, filtered and concentrated. Purification via silica gel chromatography (EtOAc/Heptanes) afforded the desired coupling product **S** (16.4 g).

### Synthesis of Intermediate T:

Disaccharide **S** (11 g) was suspended in THF: MeOH (200 mL total, 10:1 v:v) and cooled to 0°C. Ammonia was bubbled through the reaction mixture for 15 min. The reaction mixture was capped and allowed to warm to room temperature with stirring over 12 h. The reaction mixture was purged with nitrogen and concentrated *in vacuo* to a thick syrup. Intermediate **T** was isolated as a mixture of α- and β- isomers and used in the next step without purification

### Synthesis of Intermediate J:

A solution of the starting sugar **T** (7 mmol) in CH₂Cl₂ (20 mL) was treated with trichloroacetonitrile (5 mL). To the reaction mixture were added 1,8-diazabicyclo[5.4.0]undec-7-ene (DBU, 0.1 mL, 0.6 mmol) dropwise. The reaction mixture was stirred at rt for 1h, then concentrated to a viscous oil. Purification via filtration through a silica gel plug pre-treated with EtOAc containing 0.1% TEA afforded the desired trichloroacetimidate product **J** (6.2 mmol).

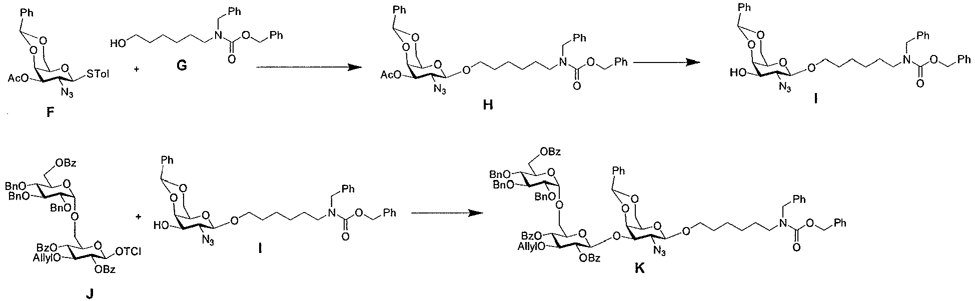

### Scheme 3. Synthesis of Key Trisaccharide Core Unit K

### Synthesis of Intermediate H:

Thioglycoside **F** (14.0 mmol) and glycosyl acceptor **G** (20.4 mmol) were combined, co-evaporated with toluene (3 x 20 mL) and dried *in vacuo* for 1h. The resulting mixture was dissolved in dry CH₂Cl₂ (130 mL) under nitrogen and the reaction mixture was cooled to -20°C. N-iodosuccinimide (28 mmol) were added followed by trifluoromethanesulfonic acid (1.4 mmol). The reaction mixture was warmed to 0°C over 10 minutes and the reaction stirred for an additional 30 minutes. The reaction mixture was quenched with triethylamine (2 mL), diluted with CH₂Cl₂ (50 mL) and washed with saturated aqueous NaHCO₃ and brine. The organic solution was dried over Na₂SO₄, filtered and concentrated. Purification via silica gel chromatography (EtOAc/Heptanes; 50-100% EtOAc gradient) afforded the desired coupling product **H** (9.2 g).

### Synthesis of Intermediate I:

Intermediate **H** (8.0 mmol) was dissolved in CH₂Cl₂ (50 mL). NaOMe (4M in MeOH, 0.1 mL, 0.4 mmol) were added and the reaction mixture was stirred at room temperature for 1h. The resulting mixture washed with water and brine. The organic solution was dried over Na₂SO₄, filtered and concentrated. Purification via silica gel chromatography (EtOAc/Heptanes; 50-100% EtOAc gradient) afforded the desired product **I** (4.5 g, 7.25 mmol).

### Synthesis of Trisaccharide K:

Glycosyl trichloroacetimidate **J** (6.0 mmol) and glycosyl acceptor **I** (7.3 mmol) were combined, co-evaporated with toluene (3 x 20 mL) and dried *in vacuo* for 1h. The resulting mixture was dissolved in dry CH₂Cl₂ (40 mL) under nitrogen and the reaction mixture was cooled to 0°C. Activated molecular sieves (AW-300, 5 g) were added and the reaction mixture was stirred for 30 min. A solution of trimethylsilyl trifluoromethanesulfonate (TMSOTf, 0.10 M in CH₂Cl₂, 3.0 mL, 0.3 mmol) was added dropwise over 10 minutes and the reaction stirred for an additional 30 minutes. The reaction mixture was quenched with triethylamine (2 mL), diluted with CH₂Cl₂ (50 mL) and washed with saturated aqueous NaHCO₃ and brine. The organic solution was dried over Na₂SO₄, filtered and concentrated. Purification via silica gel chromatography (EtOAc/Heptanes; 50-100% EtOAc gradient) afforded the desired coupling product **K** (8.2 g).

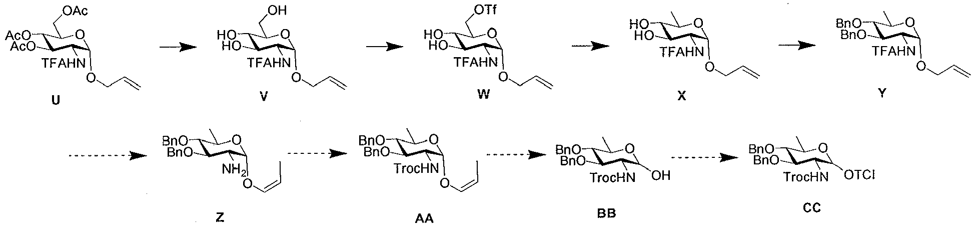

### Scheme 4. Synthesis QuiNAc Building Block CC

The following protocols describe the synthesis of intermediate **Y,** a compound that may be useful for the production of the target building block **CC.**

### Synthesis of Intermediate Y:

Known compound **V** (Christ, W. et al PCT/US96/09578)) (73 mmol) was co-evaporated with THF (3 x 100 mL) and dissolved in THF (200 mL). 2,6-lutidine (25 mL, 109 mmol) was added and the reaction mixture was cooled to - 78°C for 30 min. Trifluoromethanesulfonic acid (18.4 mL, 109 mmol) was added dropwise and the reaction mixture was stirred at -78°C for 30 min. The reaction mixture was diluted with THF (200 mL) and washed with brine (500 mL). The organics were dried over Na₂SO₄, filtered and concentrated. Intermediate **W** was used immediately in the next step without purification. Acetonitrile (100 mL) were added to the crude intermediate **W** and the reaction mixture was cooled to 0°C. NaBH₄ (9g total, 240 mmol) was added in 1 g portions allowing for bubbling to slow prior to addition of each portion. The reaction mixture was warmed to room temperature over 1 h then quenched with water (20 mL, dropwise). The suspension was concentrated *in vacuo,* suspended in EtOAc (200 mL) and filtered through celite. Product **X** was used in the next step without purification.

### Synthesis of Intermediate Y:

Intermediate **X** (8 mmol) was co-evaporated with toluene (3 x 100 mL) and dissolved in NMP:THF (20 mL total, 4:1 v:v). The reaction mixture was cooled to -0°C for 30 min and purged with nitrogen. NaH (1.05 g, 60% in mineral oil, 25 mmol) was added and the reaction mixture was stirred at -0°C for 30 min. Benzyl bromide (2.2 mL, 18.4 mmol) were added and the reaction mixture was warmed to room temperature over 1h. The reaction mixture was quenched with methanol (5 mL), diluted with EtOAc (200 mL) and washed with water (2 x200 mL). The organics were dried over Na₂SO₄, filtered and concentrated. Purification via silica gel chromatography (EtOAc/Heptanes) afforded product **Y** (2.3 g).

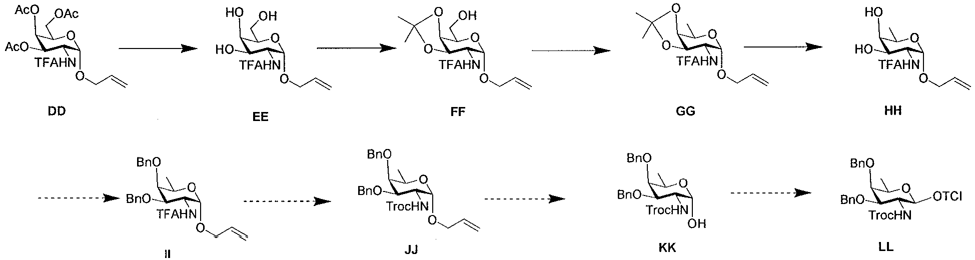

### Scheme 5. Synthesis of FucNAc Building Block LL

Compound **HH** can be prepared according to the methods of Paulsen, H. et al Liebigs Ann. Chem. 1992, 735 and is a key intermediate that may be used for the synthesis of building block **LL.**

It is intended that the foregoing detailed description be regarded as illustrative rather than limiting, and that it be understood that it is the following claims, including all equivalents, that are intended to define the scope of this invention.

## Claims

1. A synthetic oligosaccharide **1a** or **1b** 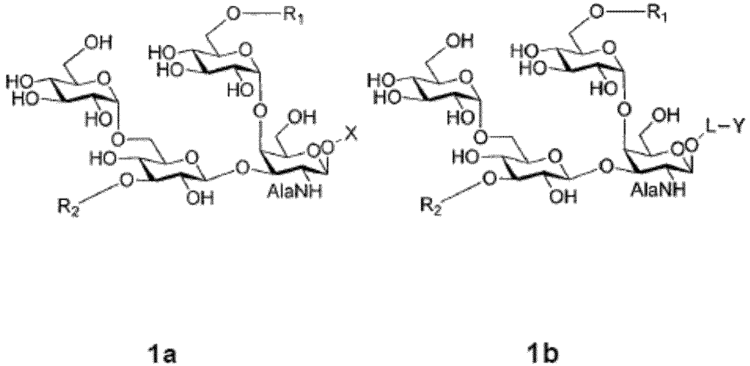 where each of R¹ and R² is independently H, a monosaccharide or an oligosaccharide, X is H, a linker group, or a protecting group; L is a linker and Y is a carrier.

2. An oligosaccharide as claimed in claim 1, wherein:
(i) each of R¹ and R² is independently selected from the group consisting of H, α-Rha-, α-Glc(1-2)-α-Rha-, β-QuiNAc(1-3)-α-Rha-, β-FucNAc(1-3)-α-Rha-, α-Rha[2,3,4- OAc]-, β-OuiNAc(1-3)-α-Rha[2,4-OAc]-, or β-FucNAc(1-3)-α-Rha[2,4,-OAc]-;
(ii) R¹ and R² is one of the combinations listed in the following table:
| **R¹** | **R²** |
|---|---|
| α-Rha- | H |
| α-Glc(1-2)-α-Rha- | H |
| H | α-Rha- |
| H | β-QuiNAc(1-3)-α-Rha- |
| H | β-FucNAc(1-3)-α-Rha- |
| H | α-Rha[2,3,4-OAc]- |
| H | β-QuiNAc(1-3)-α-Rha[2,4-OAc]- |
| H | β-FucNAc(1-3)-α-Rha[2,4,-OAc]- |
(iii) oligosaccharide 1a has formula (2a): 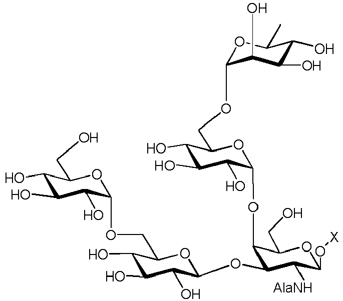 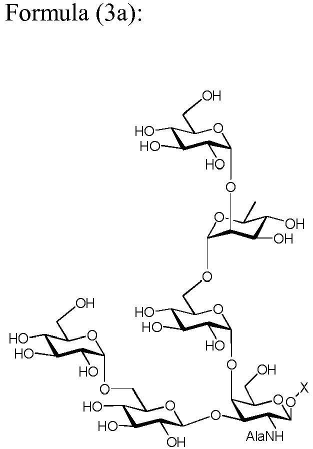 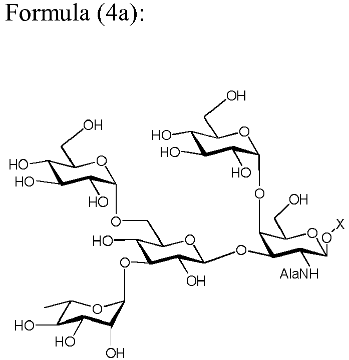 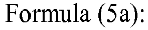 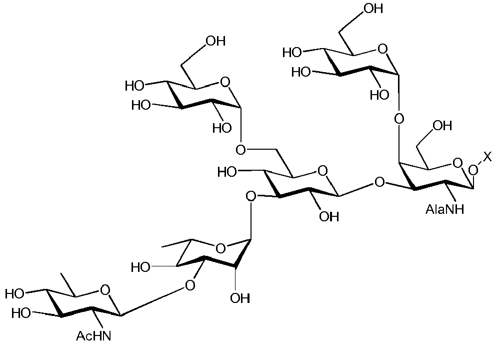 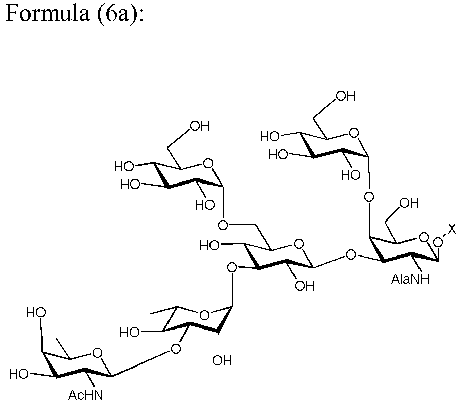 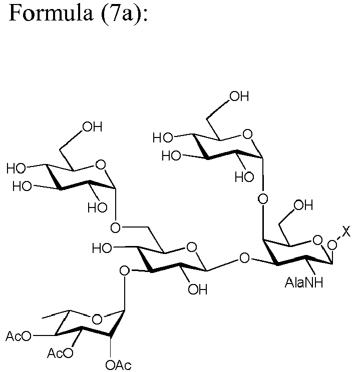 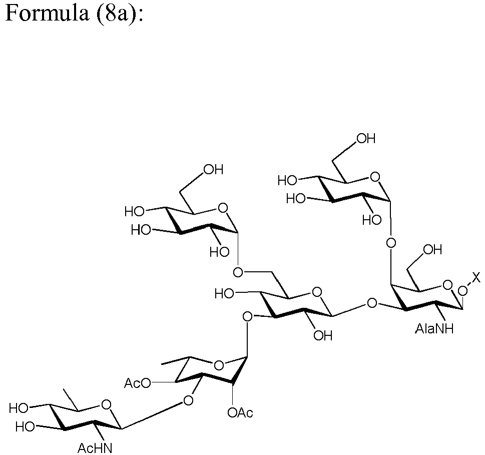 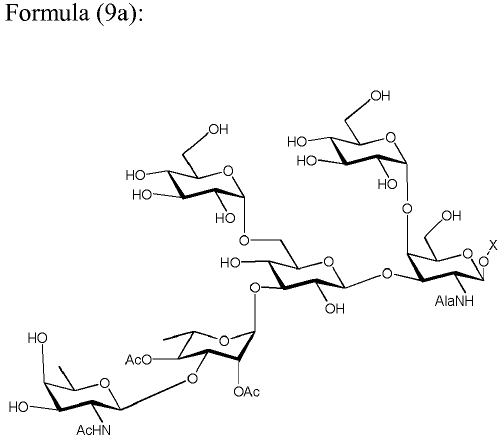 or
(iv) oligosaccharide 1b has formula (2b):

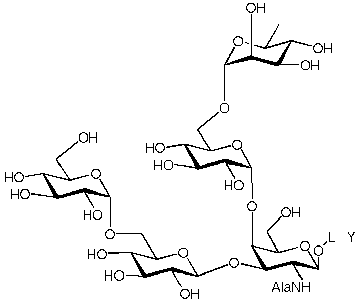

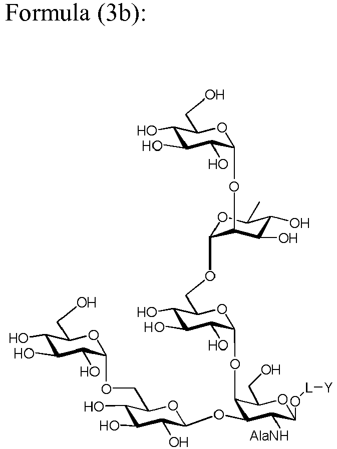

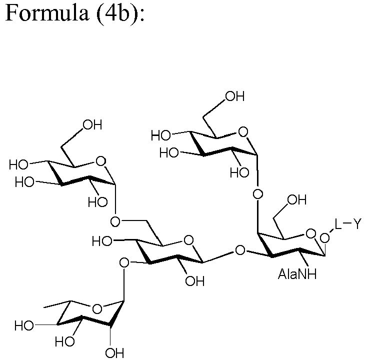

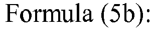

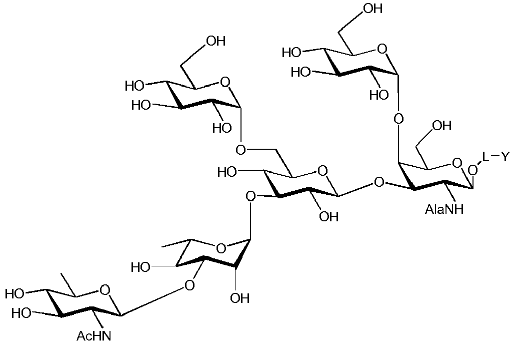

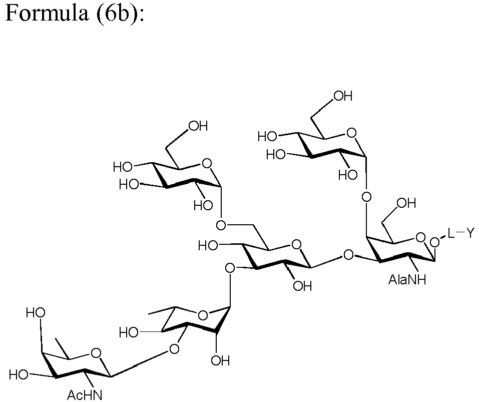

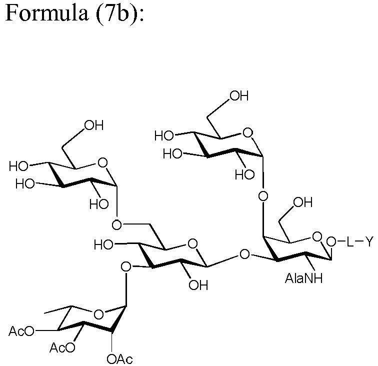

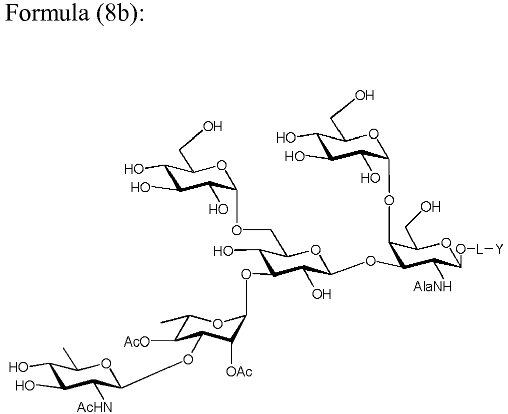

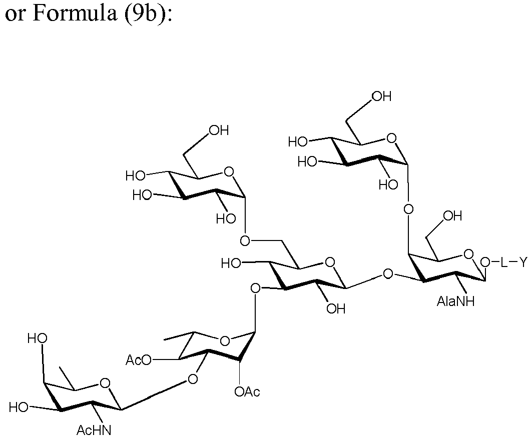

3. An oligosaccharide as claimed in claim 1 or claim 2, where:
(i) L is an alkylenethiol linker;
(ii) Y is a carrier selected from the group consisting of a protein, a peptide, a lipid, a polymer, a dendrimer, a virosome, a virus-like particle, or a combination thereof;
optionally, wherein the carrier is a carrier protein; preferably selected from the group consisting of a bacterial toxoid, a toxin, an exotoxin, and a nontoxic derivative thereof

4. An oligosaccharide as claimed in claim 3, wherein the carrier protein is selected from the group consisting of tetanus toxoid, tetanus toxin Fragment C, diphtheria toxoid, CRM, cholera toxoid, a *Staphylococcus aureus* exotoxins or toxoids, *Escherichia coli* heat labile enterotoxin, *Pseudomonas aeruginosa* exotoxin A, a genetically detoxified variants thereof, a bacterial outer membrane proteins, serotype B outer membrane protein complex (OMPC), outer membrane class 3 porin (rPorB), a porin, keyhole limpet hemocyanine (KLH), hepatitis B virus core protein, thyroglobulin, an albumin, ovalbumin, pneumococcal surface protein A (PspA), pneumococcal adhesin protein (PsaA), a purified protein derivative of tuberculin (PPD), a transferrin binding proteins, a peptidyl agonists of TLR-5, or a derivatives and/or a combinations of the above carriers;
optionally, wherein the carrier protein is selected from the group consisting of CRM 197, *Neisseria meningitidis,* bovine serum albumin (BSA), human serum albumin (HSA), poly(lysine:glutamic acid), flagellin of motile bacteria, or a derivative and/or a combinations thereof, e.g., tetanus toxoid, CRM197 or OMPC.

5. A composition comprising a synthetic oligosaccharide of any of claims 1 to4 and a pharmaceutically acceptable vehicle, optionally, wherein the composition
(i) comprises a plurality of different oligosaccharides, where each oligosaccharide is an oligosaccharide of formula **1a** or **1b;**
(ii) further comprises an adjuvant, e.g., an adjuvant selected from the group consisting of an aluminum salt, RIBI, a toll-like receptor agonists, AS01, AS02, AS03, AS04, AS05, CpG-oligodeoxynucleotide, MF-59, Montanide ISA-51 VG, Montanide ISA-720, Quil A, QS21, synthetic saponins, an immunostimulating complexes, stearyi tyrosine, a virus-like particles, a reconstituted influenza virosomes, a cytokines, mast cell activator compound 48/80, a liposomes, a muramyl dipeptides, SAF-1, or a combinations thereof; and/or
(iii) comprises an amount of at least one oligosaccharide sufficient to confer immunity against *Pseudomonas.*

6. A composition comprising an oligosaccharide of any of claims 1 to 4 for use as a vaccine.

7. A pharmaceutical composition comprising an oligosaccharide of any of claims 1 to 4 for use in inducing an immune response, e.g., an antigen-specific immune response, against *Pseudomonas* in a subject; optionally wherein the pharmaceutical composition further comprises an adjuvant.

8. An antibody preparation against an oligosaccharide according to any of claims 1 to 4, wherein the antibody preparation comprises at least one member from a polyclonal antibody, a monoclonal antibody, a mouse monoclonal IgG antibody, a humanized antibody, a chimeric antibody, a single chain antibodies, a fragment thereof, or a combination thereof.

9. A pharmaceutical composition comprising an antibody preparation of claim 8 for use in providing passive immunity against *Pseudomonas* in a subject.

10. An oligosaccharide of any of claims 1 to 4 or an antibody thereto for use in treating or preventing a disease associated with *P. aeruginosa* infection in a subject, *e.g.,* a human.

11. A method for producing an antibody, e.g., a monoclonal antibody, against an oligosaccharide according to any of claims 1 to 4, the method comprising:
(a) administering to a non-human subject, e.g., a rabbit, an effective amount of at least one oligosaccharide of any of claims 1 to 4; and
(b) isolating an antibody against an oligosaccharide according to any of claims 1 to 4, from the subject;
optionally, further comprising:
(i) administering to the subject an adjuvant; and/or
(ii) fusing a cell that produces an antibody against an oligosaccharide according to any of claims 1 to 4, from the subject to a myeloma cell and harvesting the antibody produced from a fusion subclone.

12. An antibody producing cell obtained by the method of claim 11, step (ii).

13. An antibody against an oligosaccharide according to any of claims 1 to 4, produced by the method of claim 11.

14. A method of detecting the presence of *Pseudomonas* in a sample, comprising contacting the sample with an antibody against an oligosaccharide of any of claims 1 to 4.

15. A kit comprising an antibody against an oligosaccharide of any of claims 1 to 4.

## Patentansprüche

1. Synthetisches Oligosaccharid **1a** oder **1b** 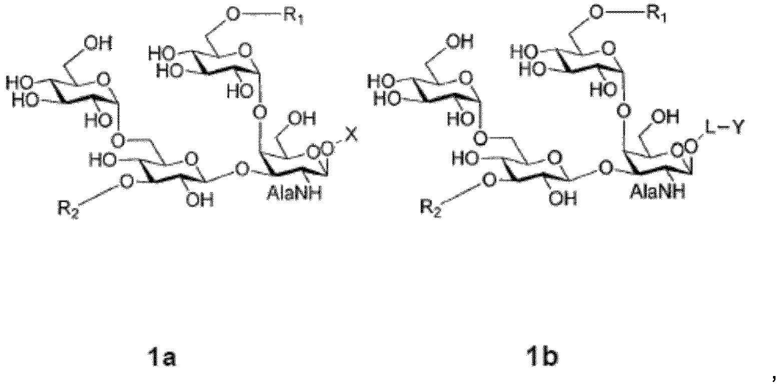 wobei jedes R¹ und R² unabhängig H, ein Monosaccharid oder ein Oligosaccharid ist, X H, eine Linker-Gruppe oder eine Schutzgruppe ist; L ein Linker ist und Y ein Träger ist.

2. Oligosaccharid nach Anspruch 1, wobei:
(i) jedes R¹ und R² unabhängig ausgewählt ist aus der Gruppe bestehend aus H, α-Rha-, α-Glc(1-2)-α-Rha-, β-QuiNAc(1-3)-α-Rha-, β-FucNAc(1-3)-α-Rha-, α-Rha[2,3,4-OAc]-, β-QuiNAc(1-3)-α-Rha[2,4-OAc]- oder β-FucNAc(1-3)-α-Rha[2,4-OAc]-;
(ii) R¹ und R² einer der in der folgenden Tabelle aufgeführten Kombinationen entsprechen:
| **R¹** | **R²** |
|---|---|
| α-Rha- | H |
| α-Glc(1-2)-α-Rha- | H |
| H | α-Rha- |
| H | β-QuiNAc(1-3)-α-Rha- |
| H | β-FucNAc(1-3)-α-Rha- |
| H | α-Rha[2,3,4-OAc]- |
| H | β-QuiNAc(1-3)-α-Rha[2,4-OAc]- |
| H | β-FucNAc(1-3)-α-Rha[2,4-OAc]- |
(iii) Oligosaccharid 1a Formel (2a) aufweist: 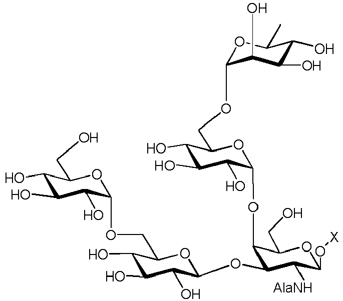 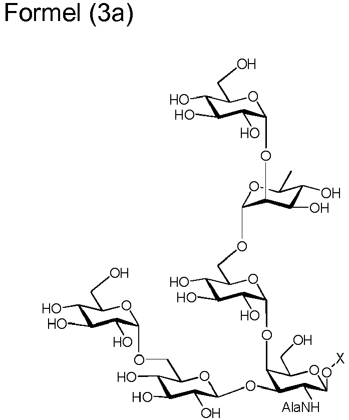 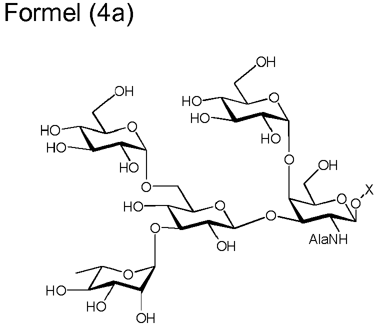 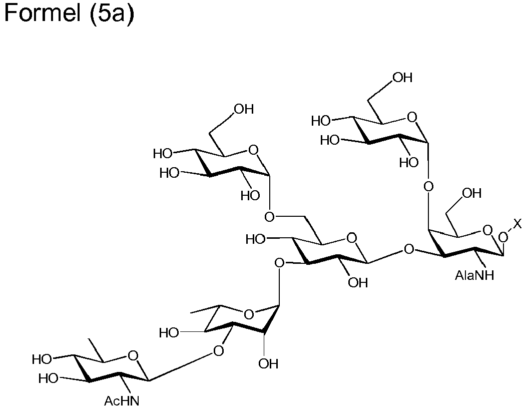 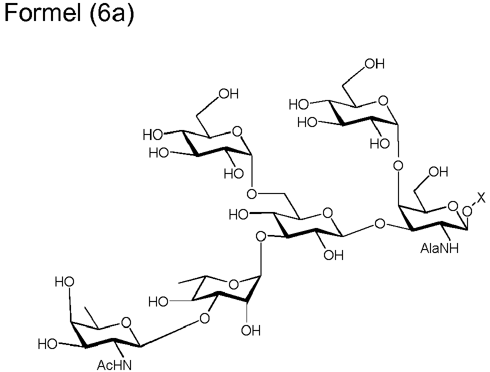 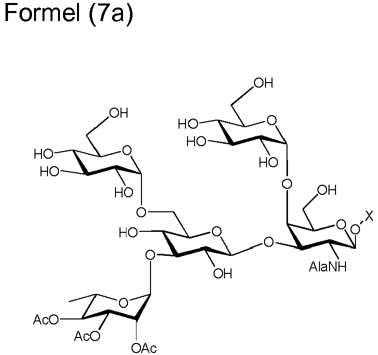 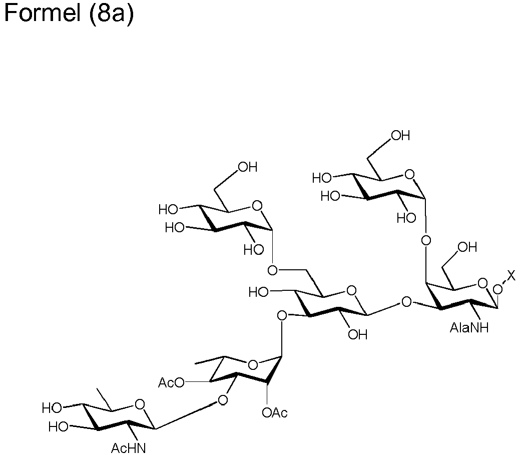 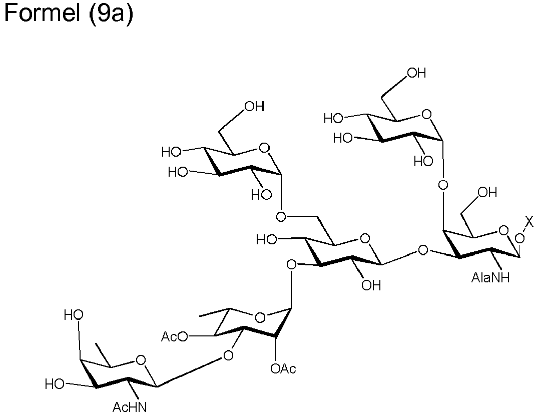 oder
(iv) Oligosaccharid 1b Formel (2b) aufweist: 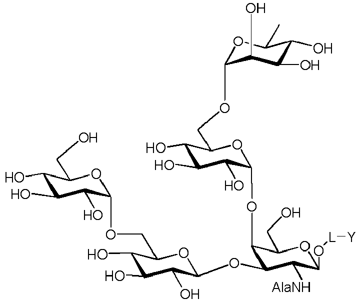 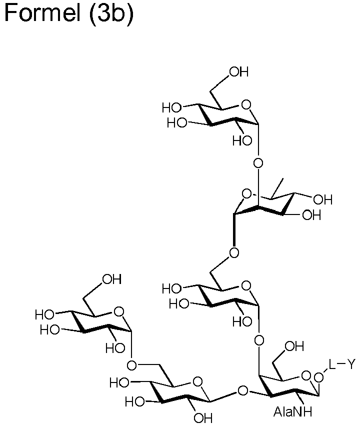 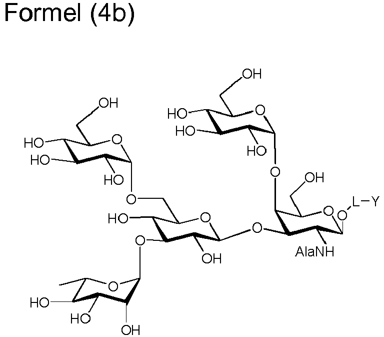 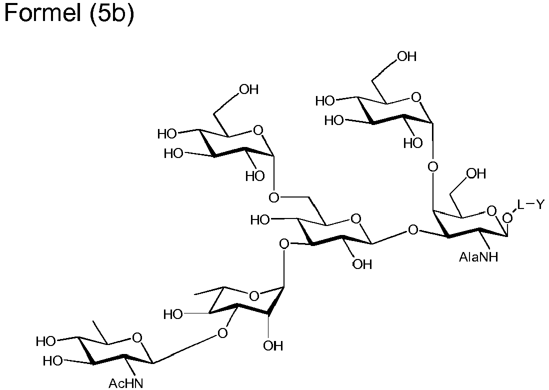 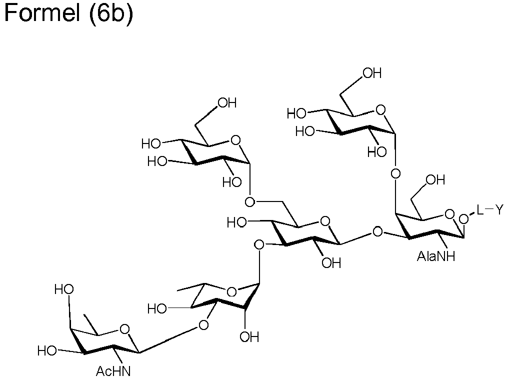 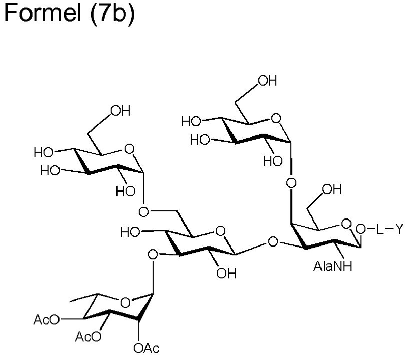 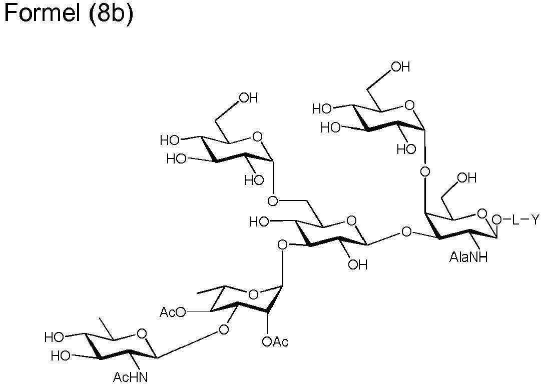 oder

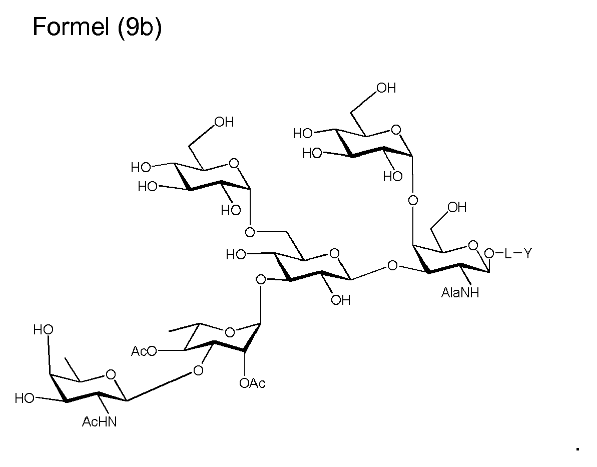

3. Oligosaccharid nach Anspruch 1 oder Anspruch 2, wobei:
(i) L ein Alkylenthiol-Linker ist;
(ii) Y ein Träger ist, der ausgewählt ist aus der Gruppe bestehend aus einem Protein, einem Peptid, einem Lipid, einem Polymer, einem Dendrimer, einem Virosom, einem Virus-ähnlichen Partikel oder einer Kombination daraus;
wobei der Träger optional ein Trägerprotein ist; vorzugsweise ausgewählt aus der Gruppe bestehend aus einem bakteriellen Toxoid, einem Toxin, einem Exotoxin und einem nichttoxischen Derivat davon.

4. Oligosaccharid nach Anspruch 3, wobei das Trägerprotein ausgewählt ist aus der Gruppe bestehend aus Tetanus-Impfstoff, Tetanustoxin Fragment C, Diphtherie-Impfstoff, CRM, Cholera-Impfstoff, *Staphylococcus* aureus-Exotoxine oder Toxoide, *Escherichia coli* hitzelabiles Enterotoxin, *Pseudomonas aeruginosa* Exotoxin A, genetisch entgifteten Varianten davon, bakteriellen Proteinen der äußeren Membran, Serotyp B äußerer Membran-Protein-Komplex (OMPC), Außenmembran-Klasse-3-Porin (rPorB), einem Porin, Schlitzschnecken-Hämocyanin (Keyhole Limpet Hemocyanin, KLH), Hepatitis B Viruskernprotein, Thyroglobulin, einem Albumin, Ovalbumin, Pneumokokken-Oberflächenprotein A (PspA), Pneumokokken-Adhäsinprotein (PsaA), einem gereinigten Proteinderivat von Tuberkulin (PPD), Transferrin-Bindeproteinen, Peptidylagonisten von TLR-5 oder Derivaten und/oder Kombinationen der vorstehend genannten Träger;
wobei das Trägerprotein optional ausgewählt ist aus der Gruppe bestehend aus CRM 197, *Neisseria meningitidis,* Bovinem Serumalbumin (BAS), Humanem Serumalbumin (HSA), Poly(lysin:Glutaminsäure), Flagellin von freibeweglichen Bakterien oder einem Derivat und/oder Kombinationen davon, wie z.B. Tetanus-Impfstoff, CRM 197 oder OMPC.

5. Zusammensetzung, die ein synthetisches Oligosaccharid nach einem der Ansprüche 1 bis 4 umfasst sowie einen pharmazeutisch verträglichen Trägerstoff, wobei die Zusammensetzung optional
(i) mehrere unterschiedliche Oligosaccharide umfasst, wobei jedes Oligosaccharid ein Oligosaccharid der Formel **1a** oder **1b** ist;
(ii) ferner einen Hilfsstoff umfasst, wie z.B. einen Hilfsstoff, der ausgewählt ist aus der Gruppe bestehend aus einem Aluminiumsalz, RIBI, einem Toll-like-Rezeptor-Agonisten, AS01, AS02, AS03, AS04, AS05, CpG-Oligodeoxynukleotid, MF-59, Montanide ISA-51 VG, Montanide ISA-720, Quil A, QS21, synthetischen Saponinen, immunstimulierenden Komplexen, Stearyl-Tyrosin, Virus-ähnlichen Partikeln, rekonstituierten Influenza-Virosomen, Zytokoninen, einer Mastzellenaktivatorverbindung 48/80, Liposomen, Myramil-Dipeptiden, SAF-1 oder Kombinationen davon; und/oder
(iii) eine Menge wenigstens eines Oligosaccharids umfasst, die ausreicht, um eine Immunität gegen *Pseudomonas* zu verleihen.

6. Zusammensetzung, die ein Oligosaccharid nach einem der Ansprüche 1 bis 4 zur Verwendung als Impfstoff umfasst.

7. Pharmazeutische Zusammensetzung, die ein Oligosaccharid nach einem der Ansprüche 1 bis 4 zur Verwendung beim Induzieren einer Immunreaktion umfasst, wie z.B. einer Antigen-spezifischen Immunreaktion gegen *Pseudomonas* in einem Subjekt; wobei die pharmazeutische Zusammensetzung optional ferner einen Hilfsstoff umfasst.

8. Antikörperzubereitung gegen ein Oligosaccharid nach einem der Ansprüche 1 bis 4, wobei die Antikörperzubereitung wenigstens ein Element aus einem polyklonalen Antikörper, einem monoklonalen Antikörper, einem monoklonalen IgG-Antikörper der Maus, einem humanisierten Antikörper, einem chimären Antikörper, einem einkettigen Antikörper umfasst, ein Fragment davon oder eine Kombination davon.

9. Pharmazeutische Zusammensetzung, die eine Antikörperzubereitung nach Anspruch 8 zur Verwendung in der Bereitstellung einer passiven Immunität gegen *Pseudomonas* in einem Subjekt umfasst.

10. Oligosaccharid nach einem der Ansprüche 1 bis 4 oder ein Antikörper dafür zur Verwendung in der Behandlung oder Prävention einer Erkrankung, die mit einer P. *aeruginosa*-Infektion in einem Subjekt, wie z.B. einem Menschen, assoziiert ist.

11. Verfahren zur Herstellung eines Antikörpers, wie z.B. eines monoklonalen Antikörpers, gegen ein Oligosaccharid nach einem der Ansprüche 1 bis 4, wobei das Verfahren folgendes umfasst:
(a) Verabreichen einer wirksamen Menge wenigstens eines Oligosaccharids nach einem der Ansprüche 1 bis 4 einem nicht-menschlichen Subjekt, wie z.B. einem Kaninchen; und
(b) Isolieren eines Antikörpers gegen ein Oligosaccharid nach einem der Ansprüche 1 bis 4 von dem Subjekt;
optional ferner folgendes umfassend:
(i) Verabreichen eines Hilfsstoffs an das Subjekt; und/oder
(ii) Fusionieren einer Zelle, die einen Antikörper gegen ein Oligosaccharid nach einem der Ansprüche 1 bis 4 erzeugt, von dem Subjekt zu einer Myelomzelle und Ernten des aus einem Fusions-Subklons erzeugten Antikörpers.

12. Antikörper, der eine Zelle erzeugt, erhalten durch das Verfahren aus Anspruch 11, Schritt (ii).

13. Antikörper gegen ein Oligosaccharid nach einem der Ansprüche 1 bis 4, erzeugt durch das Verfahren aus Anspruch 11.

14. Verfahren zum Nachweisen der Präsenz von *Pseudomonas* in einer Probe, wobei das Verfahren das Inkontaktbringen der Probe mi einem Antikörper gegen ein Oligosaccharid nach einem der Ansprüche 1 bis 4 umfasst.

15. Kit, der einen Antikörper gegen ein Oligosaccharid nach einem der Ansprüche 1 bis 4 umfasst.

## Revendications

1. Oligosaccharide de synthèse 1a or 1b 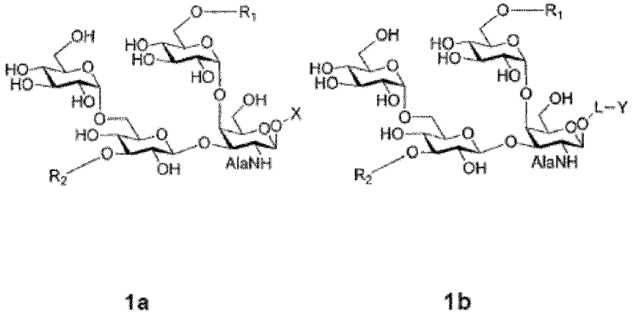 5 chacun de R¹ et de R² étant indépendamment H, un monosaccharide ou un oligosaccharide, X étant H, un groupe de liaison, ou un groupe de protection ; L étant un lieur et Y étant un vecteur.

2. Oligosaccharide selon la revendication 1 :
(i) chacun de R¹ et de R² étant indépendamment choisi dans le groupe constitué par H, α-Rha-, α-Glc(1-2)-α-Rha-, β-QuiNAc(1-3)-α-Rha-, β-FucNAc(1-3)-α-Rha-, α-Rha[2,3,4-OAc]-, β-QuiNAc(1-3)-α-Rha[2,4-OAc]-, ou β-FucNAc(1-3)-α-Rha[2,4,-OAc]-;
(ii) R¹ et R² étant l'une des combinaisons énumérées dans le tableau suivant :
| R¹ | R² |
|---|---|
| α-Rha- | H |
| α*-*Glc(1-2)-α-Rha- | H |
| H | α-Rhα- |
| H | β-QuiNAc(1-3)-α-Rha- |
| H | β-FucNAc(1-3)-α-Rha- |
| H | α-Rha[2,3,4-OAc]- |
| H | β-QuiNAc(1-3)-α-Rha[2,4-OAc]- |
| H | β-FucNAc(1-3)-α-Rha[2,4,-OAc]- |
(iii) l'oligosaccharide la ayant la formule (2a) : 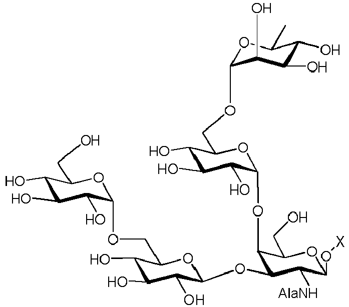 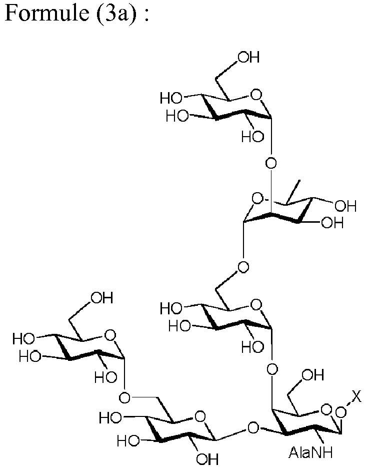 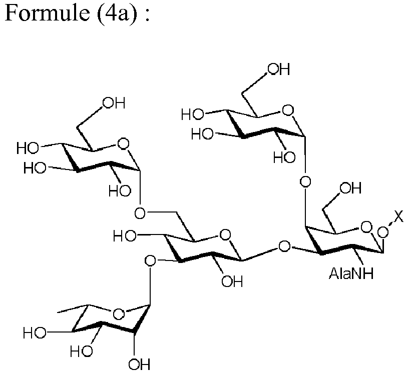 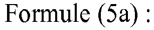 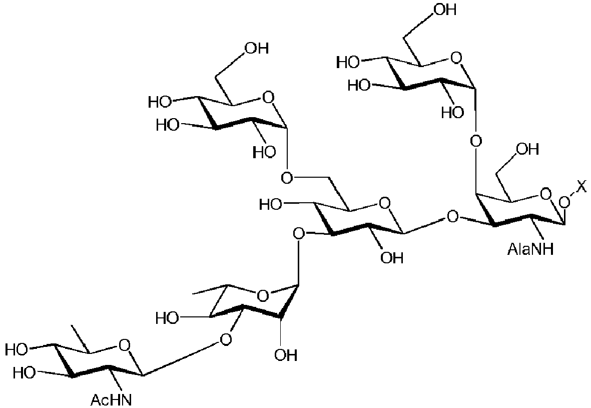 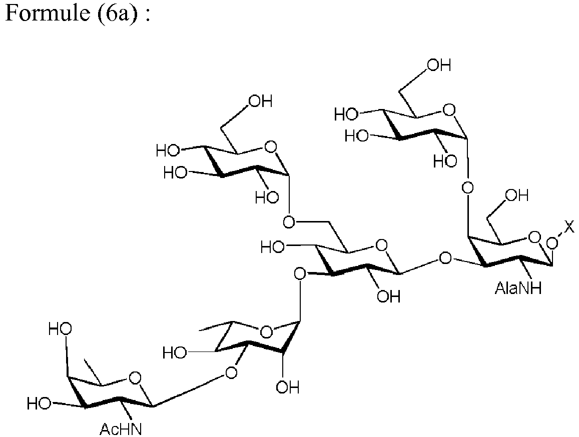 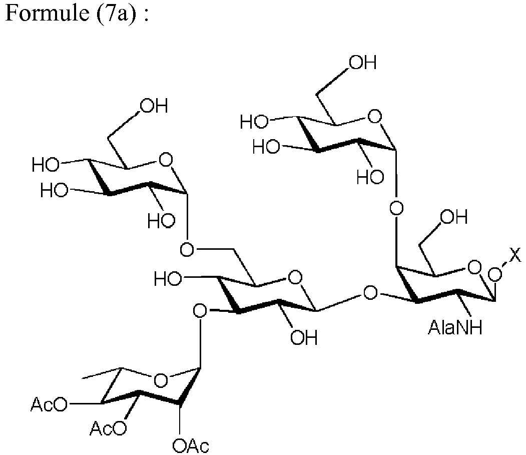 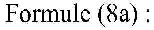 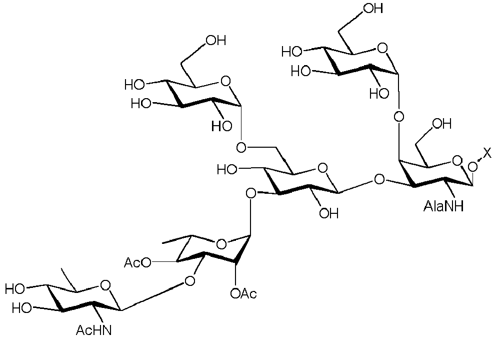 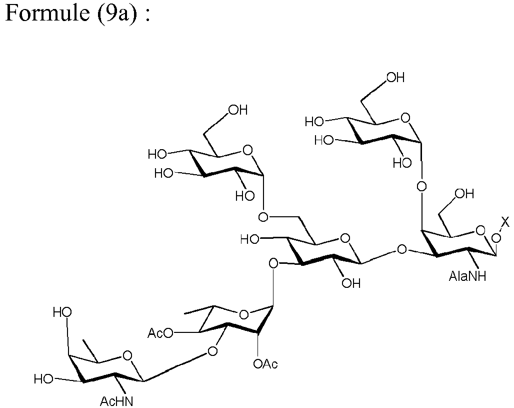 ou
(iv) l'oligosaccharide 1b ayant la formule (2b) : 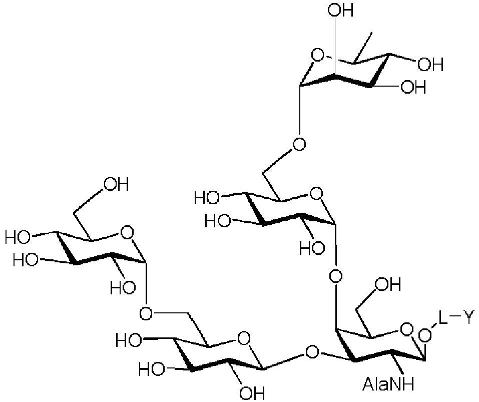 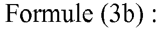 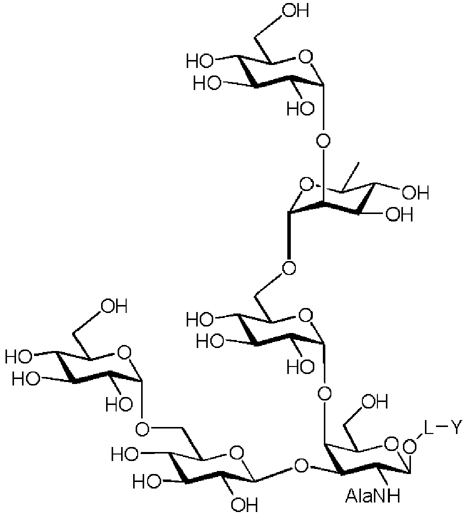 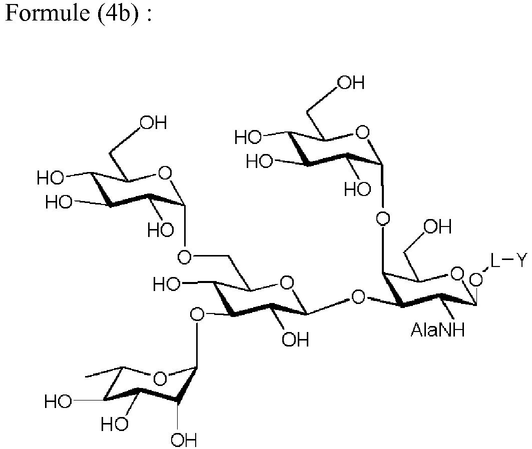 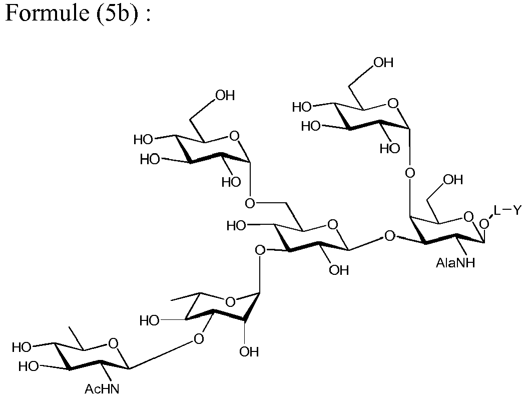 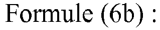 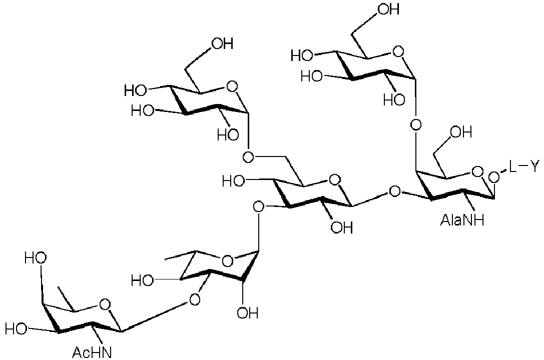 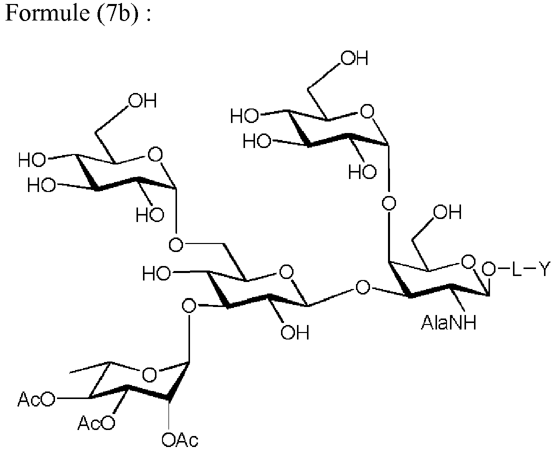 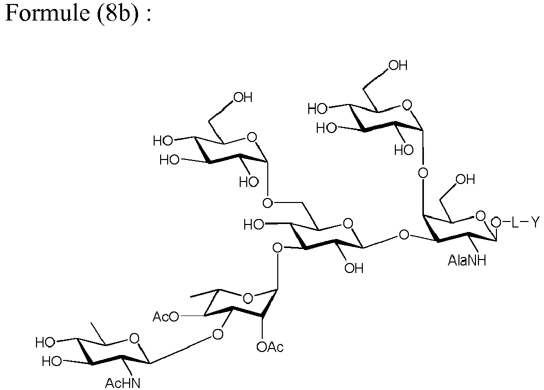 ou

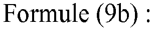

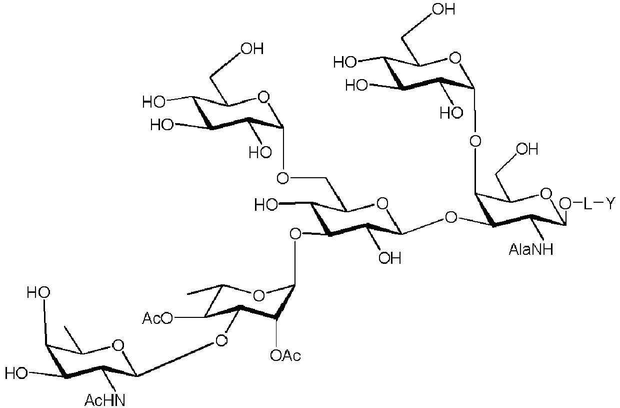

3. Oligosaccharide selon la revendication 1 ou 2 :
(i) L étant un lieur alkylènethiol ;
(ii) Y étant un vecteur choisi dans le groupe constitué par une protéine, un peptide, un lipide, un polymère, un dendrimère, un virosome, une pseudo-particule virale ou une combinaison associée ;
éventuellement, le vecteur étant une protéine porteuse ; de préférence choisie dans le groupe constitué par une anatoxine bactérienne, une toxine, une exotoxine et un dérivé non toxique associé.

4. Oligosaccharide selon la revendication 3, la protéine porteuse étant choisie dans le groupe constitué par une anatoxine tétanique, un fragment C de toxine tétanique, une anatoxine diphtérique, CRM, une anatoxine cholérique, une exotoxine ou anatoxine *Staphylococcus aureus,* une entérotoxine labile à la chaleur *Escherichia coli,* une exotoxine A *Pseudomonas aeruginosa,* une variante génétiquement détoxifiée associée, une protéine de membrane externe bactérienne, un complexe de protéines de membrane externe (OMPC) de sérotype B, une porine de classe 3 de membrane externe (rPorB), une porine, une hémocyanine de patelle (KLH), une protéine du noyau du virus de l'hépatite B, une thyroglobuline, une albumine, une ovalbumine, une protéine A de surface du pneumocoque (PspA), une protéine d'adhésine pneumococcique (PsaA), un dérivé protéique purifié de la tuberculine (PPD), une protéine de liaison à la transferrine, un agoniste peptidyl de TLR-5, ou des dérivés et/ou combinaisons des vecteurs ci-dessus ;
éventuellement, la protéine porteuse étant choisie dans le groupe constitué par CRM 197, *Neisseria meningitidis,* albumine de sérum bovin (BSA), albumine de sérum humain (HSA), poly(lysine:acide glutamique), flagelline de bactéries mobiles, ou un dérivé et/ou une combinaison associés, par exemple, anatoxine tétanique, CRM197 ou OMPC.

5. Composition comprenant un oligosaccharide de synthèse selon l'une quelconque des revendications 1 à 4 et un véhicule pharmaceutiquement acceptable, éventuellement, la composition
(i) comprenant une pluralité d'oligosaccharides différents, chaque oligosaccharide étant un oligosaccharide de formule 1a ou 1b;
(ii) comprenant en outre un adjuvant, par exemple, un adjuvant choisi dans le groupe constitué par un sel d'aluminium, RIBI, un agoniste de récepteur de type Toll, AS01, AS02, AS03, AS04, AS05, CpG-oligodéoxynucléotide, MF-59, Montanide ISA-51 VG, Montanide ISA-720, Quil A, QS21, des saponines de synthèse, un complexe immunostimulant, une stéaryl tyrosine, une pseudo-particule virale, un virosome grippal reconstitué, une cytokine, un composé activateur de mastocytes 48/80, un liposome, un dipeptide de muramyl, SAF-1 ou une combinaison associée; et/ou
(iii) comprenant une quantité d'au moins un oligosaccharide suffisante pour conférer l'immunité contre *Pseudomonas.*

6. Composition comprenant un oligosaccharide selon l'une quelconque des revendications 1 à 4 destinée à être utilisée comme un vaccin.

7. Composition pharmaceutique comprenant un oligosaccharide selon l'une quelconque des revendications 1 à 4, destinée à être utilisée pour induire une réponse immunitaire, par exemple une réponse immunitaire spécifique de l'antigène, contre *Pseudomonas* chez un sujet ; éventuellement, la composition pharmaceutique comprenant en outre un adjuvant.

8. Préparation d'anticorps dirigée contre un oligosaccharide selon l'une quelconque des revendications 1 à 4, la préparation d'anticorps comprenant au moins un élément d'un anticorps polyclonal, d'un anticorps monoclonal, d'un d'anticorps IgG monoclonal de souris, d'un anticorps humanisé, d'un anticorps chimérique, d'un anticorps à chaîne unique, d'un fragment associé, ou une combinaison associée.

9. Composition pharmaceutique comprenant une préparation d'anticorps selon la revendication 8 destinée à être utilisée pour fournir une immunité passive contre *Pseudomonas* chez un sujet.

10. Oligosaccharide selon l'une des revendications 1 à 4 ou anticorps associé destiné à être utilisé pour traiter ou empêcher une maladie associée à une infection à *P. aeruginosa* chez un sujet, par exemple un être humain.

11. Procédé de production d'un anticorps, par exemple un anticorps monoclonal, contre un oligosaccharide selon l'une quelconque des revendications 1 à 4, le procédé comprenant les étapes consistant à :
(a) administrer à un sujet non humain, par exemple un lapin, une quantité efficace d'au moins un oligosaccharide selon l'une quelconque des revendications 1 à 4 ; et
(b) isoler un anticorps dirigé contre un oligosaccharide selon l'une quelconque des revendications 1 à 4, du sujet ;
éventuellement, comprenant en outre les étapes consistant à :
(i) administrer au sujet un adjuvant ; et/ou
(ii) fusionner une cellule qui produit un anticorps dirigé contre un oligosaccharide selon l'une quelconque des revendications 1 à 4, du sujet avec une cellule myélomateuse et récolter l'anticorps produit à partir d'un sous-clone de fusion.

12. Cellule productrice d'anticorps obtenue par le procédé selon la revendication 11, étape (ii).

13. Anticorps dirigé contre un oligosaccharide selon l'une quelconque des revendications 1 à 4, produit par le procédé selon la revendication 11.

14. Procédé de détection de la présence de *Pseudomonas* dans un échantillon, comprenant l'étape consistant à mettre en contact l'échantillon avec un anticorps dirigé contre un oligosaccharide selon l'une quelconque des revendications 1 à 4.

15. Kit comprenant un anticorps dirigé contre un oligosaccharide selon l'une quelconque des revendications 1 à 4.
